# FASCICULE DE BREVET EUROPEEN

(11) **EP 1 593 682 B1**
(45) Date de publication et mention de la délivrance du brevet: **07.11.2007**
(21) Numéro de dépôt: 05290599.9
(22) Date de dépôt: 18.03.2005
(51) Int. Cl.: C07D 487/04, A61K 31/498, A61P 9/10

(54) **Dérivés de 4-oxo-4,6,7,8-tetrahydropyrrolo¬1,2-a pyrazine-6-carboxamides, leur procédé de préparation et les compositions pharmaceutiques qui les contiennent**
4-Oxo-4,6,7,8-tetrahydropyrrolo¬1,2-a pyrazin-6-carboxamid-Derivate, Vefahren zu deren Herstellung sowie diese enthaltende pharmazeutische Zusammensetzungen
Derivatives of 4-oxo-4,6,7,8-tetrahydropyrrolo¬1,2-a pyrazine-6-carboxamides, process for their preparation and pharmaceutical compositions thereof

(30) Priorité: 19.03.2004 FR 0402841
(43) Date de publication de la demande: 09.11.2005
(73) Titulaire: Les Laboratoires Servier, 92415 Courbevoie Cédex (FR)
(72) Inventeur: De Nanteuil, Guillaume, 92150 Suresnes (FR); Gloanec, Philippe, 78160 Marly Le Roi (FR); Parmentier, Jean-Gilles, 92130 Issy Les Moulineaux (FR); Benoist, Alain, 95130 Franconville (FR); Rupin, Alain, 37510 Savonnieres (FR); Vallez, Marie-Odile, 93100 Montreuil (FR); Verbeuren, Tony, 78540 Vernouillet (FR)

(56) Documents cités:
- EP-A- 0 672 658
- EP-A- 1 069 132
- EP-A- 1 215 213
- WO-A-00/75134
- WO-A-97/30708

## Description

La présente invention concerne de nouveaux dérivés de 4-oxo-4,6,7,8-tétrahydropyrrolo[1,2-a]pyrazine-6-carboxamides, leur procédé de préparation, les compositions pharmaceutiques qui les contiennent ainsi que leur utilisation en tant qu'inhibiteurs de thrombine.

La thrombine est l'enzyme clé de la coagulation et joue un rôle central dans la pathologie des thromboses veineuses et artérielles, en raison notamment de son fort pouvoir d'auto-amplification de la cascade de la coagulation (F. Toti et coll., Sang, Thrombose, Vaisseaux 1992, 4, 483-494 et T.M. Reilly et coll., Blood Coagulation and Fibrinolysis 1992, 3, 513-517).

L'inhibition directe et spécifique de la thrombine est plus efficace et présente moins de risques d'hémorragie que le traitement par l'héparine. Il existe actuellement des inhibiteurs directs de thrombine, mais ces substances peptidiques présentent l'inconvénient de ne pas être actives par voie orale.

Des dérivés peptidomimétiques, présentant une activité anti-thrombotique orale, ont déjà été décrits dans la littérature. C'est le cas notamment des dérivés de l'acide boronique décrits dans les brevets EP 293 881, EP 471 651, EP 615 978 et EP 792 883 et des dérivés décrits dans les brevets WO 94 29336, WO 95 23609 et EP 1 069 132.

Le problème de la présente invention était d'obtenir de nouveaux inhibiteurs de thrombine actifs par voie orale, à la fois bien absorbés, puissants, sélectifs et sûrs d'emploi.
A cet égard, il est intéressant d'avoir des composés présentant peu de risques d'interaction alimentaire ou médicamenteuse.

Plus spécifiquement, la présente invention concerne les composés de formule (I) : 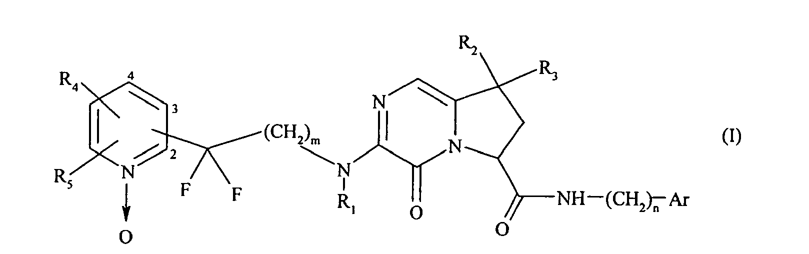 dans laquelle :
* 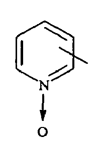 représente un groupement 1-oxydo-pyridinyle substitué par le reste de la molécule en l'une quelconque des positions 2, 3 ou 4,
* m et n, identiques ou différents, représentent chacun un entier compris entre 1 et 3,
* R₁ représente un atome d'hydrogène ou un groupement alkyle (C₁-C₆) linéaire ou ramifié,
* R₂ et R₃, identiques ou différents, représentent chacun un atome ou groupement choisi parmi les atomes d'hydrogène et d'halogène et les groupements alkyle (C₁-C₆) linéaire ou ramifié, hydroxy, acyloxy (C₁-C₆) linéaire ou ramifié et alkoxy (C₁-C₆) linéaire ou ramifié, ou forment ensemble, avec l'atome de carbone qui les porte, un cycloalcane ayant de 3 à 6 atomes de carbone,
* R₄ et R₅ représentent chacun un atome d'hydrogène, ou sont adjacents et forment ensemble, avec les atomes de carbone qui les portent, un cycle benzo,
* Ar représente un groupement aryle ou hétéroaryle,
leurs énantiomères, ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthanesulfonique, benzènesulfonique, camphorique.

Par groupement aryle, on entend phényle, biphénylyle ou naphtyle, chacun de ces groupements étant éventuellement substitué par un ou plusieurs groupements, identiques ou différents, choisis parmi :
- halogène,
- alkyle (C₁-C₆) linéaire ou ramifié éventuellement substitué par un groupement hydroxy, carboxy ou carbamoyle, le groupement carbamoyle étant lui-même éventuellement substitué par un ou deux groupements alkyle (C₁-C₆) linéaire ou ramifié,
- alkoxy (C₁-C₆) linéaire ou ramifié,
- hydroxy,
- trihalogénoalkyle (C₁-C₆) linéaire ou ramifié,
- amino éventuellement substitué par un ou deux groupements alkyle (C₁-C₆) linéaire ou ramifié,
- carboxyméthoxy,
- et carbamoylméthoxy éventuellement N-substitué par un ou deux groupements choisis parmi alkyle (C₁-C₆) linéaire ou ramifié, hydroxyalkyle (C₁-C₆) linéaire ou ramifié, alkoxyalkyle où les parties alkoxy et alkyle sont chacune en (C₁-C₆) linéaire ou ramifié, et pyridylalkyle où la partie alkyle est en (C₁-C₆) linéaire ou ramifié.

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi :
- halogène,
- alkyle (C₁-C₆) linéaire ou ramifié éventuellement substitué par un groupement hydroxy, carboxy ou carbamoyle, le groupement carbamoyle étant lui-même éventuellement substitué par un ou deux groupements alkyle (C₁-C₆) linéaire ou ramifié,
- hydroxy,
- oxo,
- alkoxy (C₁-C₆) linéaire ou ramifié,
- trihalogénoalkyle (C₁-C₆) linéaire ou ramifié,
- amino éventuellement N-substitué par un ou deux groupements alkyle (C₁-C₆) linéaire ou ramifié,
- carboxyméthoxy,
- et carbamoylméthoxy éventuellement N-substitué par un ou deux groupements choisis parmi alkyle (C₁-C₆) linéaire ou ramifié, hydroxyalkyle (C₁-C₆) linéaire ou ramifié, alkoxyalkyle où les parties alkoxy et alkyle sont chacune en (C₁-C₆) linéaire ou ramifié, et pyridylalkyle où la partie alkyle est en (C₁-C₆) linéaire ou ramifié.

Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, pyrimidinyle, pyrazinyle, pyridazinyle, indolyle, benzofuryle et quinolyle.

m est préférentiellement égal à 1.

n est préférentiellement égal à 1.

R₁, R₂ et R₃ représentent chacun préférentiellement un atome d'hydrogène.

Un aspect avantageux de l'invention concerne les composés de formule (I) pour lesquels R₄ et R₅ représentent chacun un atome d'hydrogène.

Un autre aspect avantageux de l'invention concerne les composés de formule (I) pour lesquels R₄ et R₅ sont adjacents et forment ensemble, avec les atomes de carbone qui les portent, un cycle benzo.

Le groupement Ar est préférentiellement un groupement phényle, thiényle ou pyridyle, chacun de ces groupements étant non substitué ou substitué par un ou plusieurs groupements, identiques ou différents, choisis parmi :
- halogène,
- alkyle (C₁-C₆) linéaire ou ramifié éventuellement substitué par un groupement hydroxy, carboxy ou carbamoyle, le groupement carbamoyle étant lui-même éventuellement substitué par un ou deux groupements alkyle (C₁-C₆) linéaire ou ramifié,
- alkoxy (C₁-C₆) linéaire ou ramifié,
- hydroxy,
- trihalogénoalkyle (C₁-C₆) linéaire ou ramifié,
- amino éventuellement substitué par un ou deux groupements alkyle (C₁-C₆) linéaire ou ramifié,
- carboxyméthoxy,
- et carbamoylméthoxy éventuellement N-substitué par un ou deux groupements choisis parmi alkyle (C₁-C₆) linéaire ou ramifié, hydroxyalkyle (C₁-C₆) linéaire ou ramifié, alkoxyalkyle où les parties alkoxy et alkyle sont chacune en (C₁-C₆) linéaire ou ramifié, et pyridylalkyle où la partie alkyle est en (C₁-C₆) linéaire ou ramifié.

Plus préférentiellement, Ar représente un groupement phényle non substitué ou substitué par un ou plusieurs atomes d'halogène, identiques ou différents, choisis parmi fluor et chlore.

Les composés préférés de formule (I) sont :
- le 3-{[2,2-difluoro-2-(1-oxydo-2-pyridyl)-éthyl]-amino}-N-(2-fluorobenzyl)-4-oxo-4,6, 7,8-tétrahydropyrrolo[1,2-a]pyrazine-6-carboxamide, ainsi que son énantiomère (6S) ;
- le 3-{[2,2-difluoro-2-(1-oxydo-2-pyridyl)-éthyl]-amino}-N-(2,6-difluorobenzyl)-4-oxo-4,6,7,8-tétrahydropyrrolo[1,2-a]pyrazine-6-carboxamide, ainsi que son énantiomère (6S) ;
- le 3-{[2,2-difluoro-2-(1-oxydo-2-pyridyl)-éthyl]-amino}-N-(2-chlorobenzyl)-4-oxo-4, 6,7,8-tétrahydropyrrolo[1,2-a]pyrazine-6-carboxamide, ainsi que son énantiomère (6S) ;
- le 3-{[2,2-difluoro-2-(1-oxydo-2-pyridyl)-éthyl]-amino}-N-(2,5-difluorobenzyl)-4-oxo-4,6,7,8-tétrahydropyrrolo[1,2-a]pyrazine-6-carboxamide, ainsi que son énantiomère (6S) ;
- le 3-{[2,2-difluoro-2-(1-oxydo-2-pyridyl)-éthyl]-amino}-N-(2,3-difluorobenzyl)-4-oxo-4,6,7,8-tétrahydropyrrolo[1,2-a]pyrazine-6-carboxamide, ainsi que son énantiomère (6S),
- et le 3-{[2,2-difluoro-2-(1-oxydo-2-pyridyl)-éthyl]-amino}-N-(2,3,6-trifluorobenzyl)-4-oxo-4,6,7,8-tétrahydropyrrolo[1,2-a]pyrazine-6-carboxamide, ainsi que son énantiomère (6S).

L'invention s'étend également au procédé de préparation des composés de formule (I) caractérisé en ce que l'on réduit un composé de formule (II) : 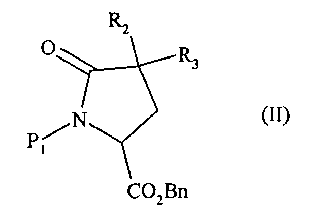 dans laquelle R₂ et R₃ sont tels que définis dans la formule (I), P₁ représente un groupement protecteur de la fonction amino et Bn représente le groupement benzyle,
à l'aide d'un agent réducteur,
pour conduire au composé de formule (III) : 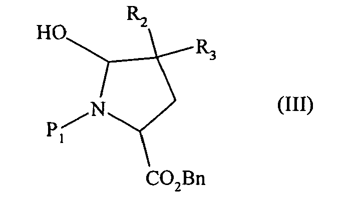 dans laquelle R₂, R₃, P₁ et Bn ont la même signification que précédemment,
dont on transforme la fonction hydroxy en méthoxy puis en fonction cyano par des réactions classiques de la chimie organique, pour conduire, après déprotection de la fonction amino, au composé de formule (IV) : 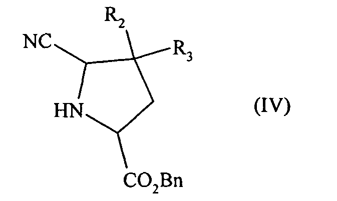 dans laquelle R₂, R₃, et Bn ont la même signification que précédemment,
que l'on met en réaction avec du chlorure d'oxalyle pour conduire au composé de formule (V) : 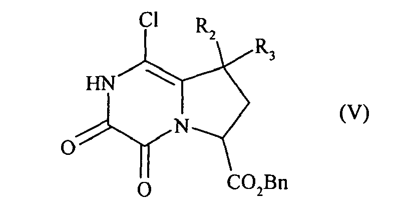 dans laquelle R₂, R₃ et Bn ont la même signification que précédemment,
que l'on soumet à une réaction d'hydrogénation catalytique, pour conduire au composé de formule (VI) : 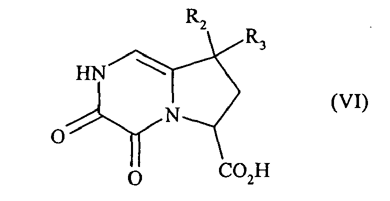 dans laquelle R₂ et R₃ sont tels que définis précédemment,
que l'on estérifie en composé de formule (VII) : 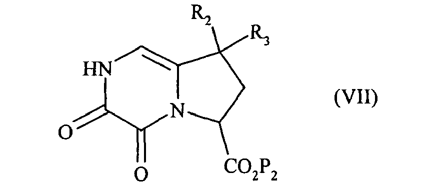 dans laquelle R₂ et R₃ sont tels que définis précédemment, et P₂ représente un groupement alkyle (C₁-C₆) linéaire ou ramifié,
que l'on met en réaction avec un agent de bromation, pour conduire au composé de formule (VIII) : 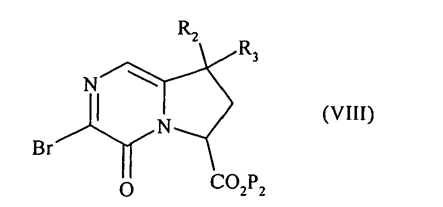 dans laquelle R₂, R₃ et P₂ sont tels que définis précédemment,
que l'on met en réaction avec la 2-mercaptopyridine, pour conduire au composé de formule (IX) : 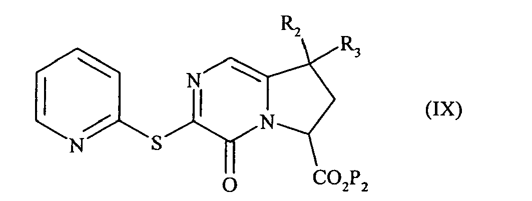 dans laquelle R₂, R₃ et P₂ sont tels que définis précédemment,
que l'on met en réaction avec le N-oxyde de formule (X) : 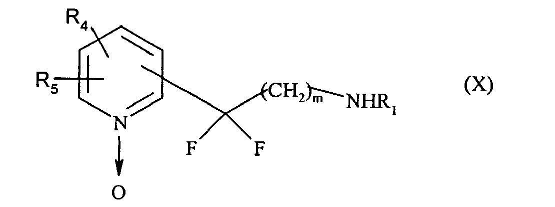 dans laquelle m, R₁, R₄ et R₅ sont tels que définis dans la formule (I),
pour conduire au composé de formule (XI) : 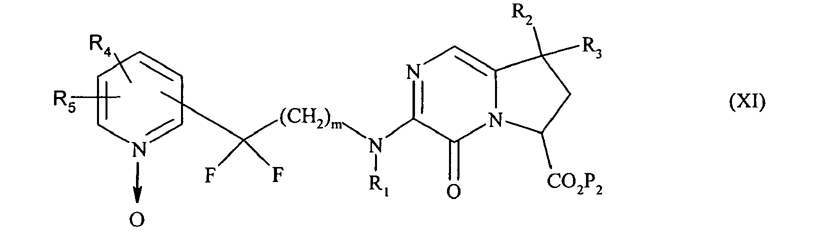 dans laquelle m, R₁, R₂, R₃ R₄, R₅ et P₂ sont tels que définis précédemment,
dont on déprotège la fonction acide, pour conduire au composé de formule (XII) : 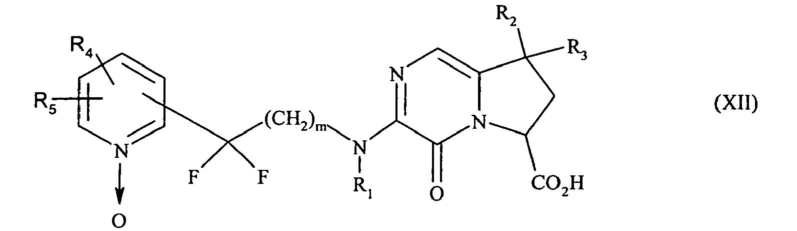 dans laquelle m, R₁, R₂, R₃ R₄ et R₅ sont tels que définis précédemment,
que l'on met en réaction avec un composé de formule (XIII) : 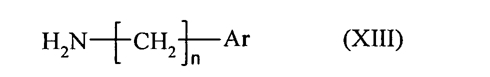 dans laquelle n et Ar ont la même signification que dans la formule (I),
en présence d'un agent de couplage,
pour conduire au composé de formule (I).

Les sels d'addition des composés de formule (I) sont obtenus par réaction du composé avec un acide pharmaceutiquement acceptable.

Les composés de formule (I) ont un centre asymétrique et peuvent donc exister sous forme de mélange racémique ou sous forme optiquement active.

Les composés de formule (I) optiquement actifs peuvent être obtenus, par exemple en partant du composé optiquement actif de formule (II) correspondant, ou par séparation du mélange racémique correspondant de formule (I), par exemple par chromatographie HPLC chirale.

Les composés de formule (I) préférés sont ceux pour lesquels la configuration du centre asymétrique en alpha de l'amide est (S).

Les composés de formule (II) sont obtenus par benzylation des acides correspondants.

Les composés de la présente invention présentent des propriétés pharmacologiques particulièrement intéressantes.

Ce sont de puissants inhibiteurs de thrombine actifs par voie orale.

Ces propriétés les rendent utiles dans le traitement des angines stables ou non, des maladies d'origine thrombotique et/ou donnant lieu à des complications thrombotiques, dans le traitement ou la prévention de l'infarctus du myocarde et des thromboses veineuses ou artérielles, ainsi que dans le traitement des complications des maladies vasculaires et cardiovasculaires telles que l'athérosclérose, l'artérite, la maladie veineuse, et dans le traitement de toutes les maladies impliquant une formation et/ou une activité de la thrombine.

Ils peuvent également être utilisés en association thérapeutique avec un thrombolytique.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention.

Les produits de départ utilisés sont des produits de départ connus ou préparés selon des modes opératoires connus.

Les structures des composés décrits dans les exemples ont été déterminées selon les techniques spectrophotométriques usuelles (infrarouge, RMN, spectrométrie de masse).

### EXEMPLE 1 : 3-{[2,2-Difluoro-2-(1-oxydo-2-pyridyl)-éthyl]-amino}-N-(2-fluorobenzyl)-4-oxo-4,6,7,8-tétrahydropyrrolo[1,2-a]pyrazine-6-carboxamide

### Stade A : N-Tert-butoxycarbonyl-5-oxoprolinate de benzyle

A 10 mmoles de 5-oxoprolinate de benzyle (dont le procédé de préparation est décrit par E. Campaigne et coll. (J. Heterocycl. Chem. 1975, 12, 391)) en solution dans le dichlorométhane sont ajoutées à 0°C 11 mmoles de diméthylaminopyridine et 11 mmoles de dicarbonate de di-tert-butyle. Après 24 heures d'agitation à température ambiante, le milieu réactionnel est lavé puis séché et évaporé pour conduire au produit attendu sous la forme d'une huile visqueuse.

### Stade B : N-Tert-butoxycarbonyl-5-hydroxy-prolinate de benzyle

A 10 mmoles du composé obtenu dans le stade précédent en solution dans le tétrahydrofurane sont ajoutées, sous argon et à -78°C, 18 mmoles d'une solution 1M d'hydrure de diisobutylaluminium dans l'hexane. Après 20 minutes d'agitation à -78°C, une solution aqueuse saturée de chlorure d'ammonium, puis une solution aqueuse de carbonate de sodium à 10 % sont ajoutées. Après 1 nuit d'agitation à température ambiante, le milieu réactionnel est filtré, le filtrat est évaporé, repris par du dichlorométhane. La phase organique est lavée, séchée puis évaporée. Le résidu est purifié par chromatographie sur gel de silice, en utilisant comme éluant un mélange dichlorométhane/acétate d'éthyle 95/5. On obtient le produit attendu sous la forme d'une huile jaune.

### Stade C : N-Tert-butoxycarbonyl-5-méthoxy-prolinate de benzyle

A 10 mmoles du composé obtenu dans le stade précédent est ajoutée une solution d'acide para-toluène sulfonique à 0,1 % dans le méthanol anhydre (88 ml). Après ½ heure d'agitation, une solution aqueuse de carbonate de sodium à 10 % est ajoutée et le produit est extrait par du dichlorométhane. On obtient le produit attendu sous la forme d'une huile légèrement jaune.

### Stade D : 5-Cyano-prolinate de benzyle, chlorhydrate

A 10 mmoles du composé obtenu dans le stade précédent sont ajoutées, à -40°C et sous argon, une solution de tétrachlorure d'étain dans le dichlorométhane anhydre à 5 % v/v (7,1 ml), puis du cyanure de triméthylsilyle (20,6 mmoles). Après 2 heures d'agitation à -40°C, une solution aqueuse de carbonate de sodium à 10 % est ajoutée, la phase aqueuse est extraite au dichlorométhane, la phase organique est lavée, séchée puis évaporée. Le résidu obtenu est purifié par chromatographie sur gel de silice en utilisant comme éluant un mélange dichlorométhane/acétate d'éthyle 95/5. L'huile jaune obtenue est mise en solution dans l'acétate d'éthyle puis on fait passer à 0°C un courant d'acide chlorhydrique gaz pendant 30 minutes. Après une nuit d'agitation à température ambiante, le précipité formé est filtré, rincé à l'acétate d'éthyle et séché sous vide au dessicateur.

### Stade E : 1-Chloro-3,4-dioxo-2,3,4,6,7,8-hexahydropyrrolo[1,2-α]pyrazine-6-carboxylate de benzyle

A 200 g du composé obtenu dans le stade précédent en solution dans le toluène est ajouté à 0°C du chlorure d'oxalyle (144 ml). Le mélange est ensuite ramené à température ambiante et agité 15 heures, puis le solvant est évaporé. Le résidu obtenu est purifié par chromatographie sur silice en utilisant comme éluant un mélange dichlorométhane/méthanol 9/1, pour conduire au produit attendu.

### Stade F : Acide 3,4-dioxo-2,3,4,6,7,8-hexahydropyrrolo[1,2-a]pyrazine-6-carboxylique

3 g du composé obtenu au stade précédent sont dissous dans 50 ml d'éthanol, puis 1,43 ml de triéthylamine sont ajoutés, suivis de 0,5 g de palladium sur charbon. On place ensuite le mélange sous atmosphère d'hydrogène, à température ambiante et pression atmosphérique, pendant 5 heures. Après filtration du catalyseur, le solvant est évaporé, pour conduire au produit attendu.

### Stade G : 3,4-Dioxo-2,3,4,6,7,8-hexahydropyrrolo[1,2-a]pyrazine-6-carboxylate d'éthyle

A une suspension de 1,83 g du composé obtenu au stade précédent dans 20 ml d'éthanol anhydre, on additionne au goutte à goutte, à 0°C, 11,83 ml de chlorure de triméthylsilyle. Le mélange réactionnel est ensuite agité à température ambiante pendant 15 heures. Le solvant est évaporé, et le résidu est repris au dichlorométhane. La phase organique est lavée, séchée, filtrée et évaporée, puis le produit brut est purifié par chromatographie sur silice (éluant : dichlorométhane/éthanol 95/5), pour conduire au produit attendu.

### Stade H : 3-Bromo-4-oxo-4,6,7,8-tétrahydropyrrolo[1,2-α]pyrazine-6-carboxylate d'éthyle

A 73 g du composé obtenu au stade précédent en suspension dans 400 ml de dichloroéthane sont ajoutés 34,7 g de phosphate de sodium dibasique (Na₂HPO₄) dans 90 ml de dichloroéthane, puis 100 g d'oxybromure de phosphore (POBr₃) dans 340 ml de dichloroéthane. Le mélange réactionnel est ensuite chauffé à 50°C pendant 16 heures, puis il est refroidi à 0°C par un bain d'eau et de glace, et 650 ml d'une solution à 10 % de carbonate de sodium sont ajoutés.
La phase organique est lavée à l'eau, puis les phases aqueuses sont réunies et extraites à l'acétate d'isopropyle. Les phases organiques réunies sont concentrées à 160 ml, puis 160 ml d'acétate d'isopropyle sont ajoutés, et le mélange est à nouveau concentré à 160 ml. Le mélange est alors placé dans un bain à 50°C et 250 ml de n-heptane sont ajoutés en 1 heure. Après 1 heure supplémentaire à 50°C, le précipité obtenu est filtré, rincé avec un mélange d'acétate d'isopropyle et de n-heptane 1/3 et séché, pour conduire au produit attendu.

### Stade I : 4-Oxo-3-(2-pyridylthio)-4,6,7,8-tétrahydropyrrolo[1,2-α]pyrazine-6-carboxylate d'éthyle

A 80,5 g du composé obtenu au stade précédent en solution dans 410 ml d'acétonitrile sont ajoutés, en trois fois à 20 minutes d'intervalle, 34,3 g de 2-mercaptopyridine. L'addition est exothermique est la température du mélange réactionnel monte à 35°C.
Le milieu devient hétérogène. Après 1h45 d'agitation, le solvant est évaporé, et le résidu est repris à l'acétate d'éthyle et à l'eau. La phase organique est lavée, séchée, filtrée et évaporée à sec. Le résidu obtenu est purifié sur colonne de chromatographie (dichlorométhane/éthanol 98/2 puis 95/5) pour conduire au produit attendu sous la forme d'une huile orange qui cristallise lentement.

### Stade J : 3-{[2,2-Difluoro-2-(1-oxydo-2-pyridyl)-éthyl]-amino}-4-oxo-4,6,7,8-tétrahydropyrrolo[1,2-α]pyrazine-6-carboxylate d'éthyle

A 4,6 g du composé obtenu au stade précédent en suspension dans 30 ml d'acétonitrile sont ajoutés 2,78 g de 2,2-difluoro-2-(1-oxydo-2-pyridyl)-éthanamine, puis 1,5 g de chlorure de zinc. Le mélange est ensuite porté au reflux pendant 15 heures. Le milieu réactionnel devient limpide. Le solvant est ensuite évaporé, et le résidu est repris au dichlorométhane. La phase organique est lavée, séchée, filtrée et évaporée, et le résidu obtenu est purifié par chromatographie sur silice (éluant : dichlorométhane/isopropanol 9/1) pour conduire au produit attendu.

### Stade K: Acide 3-{[2,2-difluoro-2-(1-oxydo-2-pyridyl)-éthyl]-amino}-4-oxo-4,6,7,8-tétrahydropyrrolo[1,2-α]pyrazine-6-carboxylique

A 9 g du composé obtenu au stade précédent en solution dans 100 ml de dioxanne et 20 ml d'eau sont ajoutés 2 équivalents de soude 1N. Après 24h à température ambiante, on acidifie par 2 équivalents d'acide chlorhydrique 1N, puis on évapore le milieu réactionnel. Le résidu est repris 2 fois par 30 ml de toluène et séché, pour conduire au produit attendu sous la forme d'un solide blanc.

### Stade L: 3-{[2,2-Difluoro-2-(1-oxydo-2-pyridyl)-éthyl]-amino}-N-(2-fluorobenzyl)-4-oxo-4,6,7,8-tétrahydropyrrolo[1,2-a]pyrazine-6-carboxamide

3 g du composé obtenu au stade précédent sont mis à réagir avec 1,18 g de 2-fluorobenzylamine en présence de 3,57 g d'hexafluorophosphate de 1-[bis(diméthylamino)-méthylène]-1H-1,2,3-triazolo[4,5-b] pyridinium 3-oxyde (HATU) et 1,64 ml de diisopropyléthylamine, dans 60 ml de diméthylformamide.
Après 15 heures d'agitation à température ambiante, le solvant est évaporé, et le résidu obtenu est repris par de l'acétate d'éthyle et de l'eau. La phase organique est lavée, séchée puis évaporée, et le résidu est purifié par chromatographie sur silice, pour conduire au produit attendu sous la forme d'un mélange racémique.

| *Microanalyse élémentaire* : | | | |
|---|---|---|---|
| | *%C* | *%H* | *%N* |
| *Calculé*: | *57,52* | *4,39* | *15,24* |
| *Trouvé*: | *57,63* | *4,18* | *15,05* |

### EXEMPLE 2 : (6R)-3-{[2,2-Difluoro-2-(1-oxydo-2-pyridyl)-éthyl]-amino}-N-(2-fluorobenzyl)-4-oxo-4,6,7,8-tétrahydropyrrolo[1,2-a]pyrazine-6-carboxamide

Le mélange racémique de l'exemple 1 est séparé par chromatographie HPLC préparative sur phase chirale (colonne Chiralpak AD, éluant acétonitrile/isopropanol/diéthylamine 500/500/1).
Le produit attendu est le premier des énantiomères ainsi obtenu.

### EXEMPLE 3 : (6S)-3-{[2,2-Difluoro-2-(1-oxydo-2-pyridyl)-éthyl]-amino}-N-(2-fluorobenzyl)-4-oxo-4,6,7,8-tétrahydropyrrolo[1,2-a] pyrazine-6-carboxamide

Le mélange racémique de l'exemple 1 est séparé par chromatographie HPLC préparative sur phase chirale (colonne Chiralpak AD, éluant acétonitrile/isopropanol/diéthylamine 500/500/1).
Le produit attendu est le deuxième des énantiomères ainsi obtenu.

*Pouvoir rotatoire : α_{D} = - 116,07° (méthanol, 20°C, c=1,4)*

### EXEMPLE 4 : (6R)-3-{[2,2-Difluoro-2-(1-oxydo-2-pyridyl)-éthyl]-amino}-N-{2-[2-(éthylamino)-2-oxoéthoxy]-benzyl}-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par le 2-[2-(aminométhyl)-phénoxy]-N-éthylacétamide, suivi de la séparation du mélange racémique ainsi obtenu par chromatographie HPLC préparative chirale (colonne Chiralpak AD, éluant acétonitrile/isopropanol/diéthylamine 500/500/1).
Le produit attendu est le premier des énantiomères ainsi obtenu.

| *Microanalyse élémentaire :* | | | |
|---|---|---|---|
| | *%C* | *%H* | *%N* |
| *Calculé*: | *57,56* | *5, 20* | *15,49* |
| *Trouvé*: | *58,11* | *5,15* | *15,55* |

*Pouvoir rotatoire : α_{D} = + 93,1° (méthanol, 20°C, c=0,7)*

### EXEMPLE 5 : (6S)-3-{[2,2-Difluoro-2-(1-oxydo-2-pyridyl)-éthyl]-amino}-N-{2-[2-(éthylamino)-2-oxoéthoxy]-benzyl}-4-oxo-4,6,7,8-tétrahydropyrrolo [1,2-a]pyrazine-6-carboxamide

Le produit attendu est le deuxième des énantiomères séparés à l'exemple 4.

*Pouvoir rotatoire : α_{D}* = - *98,8° (méthanol, 20°C, c=0,8)*

### EXEMPLE 6 : 3-{[2,2-Difluoro-2-(1-oxydo-2-pyridyl)-éthyl]-amino}-N-(2,4-difluorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2,4-difluorobenzylamine.

*Spectrométrie de masse ESI (acétonitrile*/*eau) :* [M+H]⁺= 478,15.

### EXEMPLE 7 : 3-{[2,2-Difluoro-2-(1-oxydo-2-pyridyl)-éthyl]-amino}-N-(2,6-difluorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2,6-difluorobenzylamine.

*Point de fusion : 227-228°C*

| *Microanalyse élémentaire :* | | | |
|---|---|---|---|
| | %C | *%H* | *%N* |
| *Calculé*: | *55,35* | *4,01* | *14,67* |
| *Trouvé*: | *55,04* | *4, 04* | *14, 28* |

### EXEMPLE 8 : 3-{[2,2-Difluoro-2-(1-oxydo-2-pyridyl)-éthyl]-amino}-N-(2-chlorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2-chlorobenzylamine.

| *Microanalyse élémentaire :* | | | | |
|---|---|---|---|---|
| | %C | *%H* | *%N* | *%Cl* |
| *Calculé*: | *55,53* | *4,24* | *14,72* | *7,45* |
| *Trouvé*: | *55,32* | *4,33* | *14,33* | *7,73* |

### EXEMPLE 9 : 3-{[2,2-Difluoro-2-(1-oxydo-2-pyridyl)-éthyl]-amino}-N-(3,4-difluorobenzyl)-4-oxo-4,6,7,8-tétrahydropyrrolo[1,2-a]pyrazine-G-carboxamide

Le produit attendu est obtenu selon le procédé décrit dans l'exemple 1, en remplaçant, au stade L, la 2-fluorobenzylamine par la 3,4-difluorobenzylamine.

*Point de fusion : 204°C*

| *Microanalyse élémentaire :* | | | |
|---|---|---|---|
| | *% C* | *% H* | *%N* |
| *Calculé*: | *55,35* | *4,01* | *14,67* |
| *Trouvé*: | *55,07* | *3,92* | *14,40* |

### EXEMPLE 10 : 3-{[2,2-Difluoro-2-(1-oxydo-2-pyridyl)-éthyl]-amino}-N-(2,5-difluorobenzyl)-4-oxo-4,6,7,8-tétrahydropyrrolo [1,2-a]pyrazine-6-carboxamide

Le produit attendu est obtenu selon le procédé décrit dans l'exemple 1, en remplaçant, au stade L, la 2-fluorobenzylamine par la 2,5-difluorobenzylamine.

*Point de fusion : 189°C*

| *Microanalyse élémentaire :* | | | |
|---|---|---|---|
| | *%C* | *%H* | *%N* |
| *Calculé*: | *55,35* | *4,01* | *14,67* |
| *Trouvé*: | *55,95* | *4,41* | *14,13* |

### EXEMPLE 11 : 3-{[2,2-Difluoro-2-(1-oxydo-2-pyridyl)-éthyl]-amino}-N-(2,6-dichtorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2,6-dichlorobenzylamine.

*Point de fusion : 122°C*

| *Microanalyse élémentaire :* | | | | |
|---|---|---|---|---|
| | %C | *% H* | *% N* | *%Cl* |
| *Calculé*: | *51,78* | *3,75* | *13,72* | *13,89* |
| *Trouvé*: | *52, 23* | *3,73* | *13,66* | *14, 06* |

### EXEMPLE 12 : 3-{[2,2-Difluoro-2-(1-oxydo-2-pyridyl)-éthyl]-amino}-N-(2-chloro-6-fluorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2-chloro-6-fluorobenzylamine.

*Point de fusion : 221°C*

| *Microanalyse élémentaire :* | | | | |
|---|---|---|---|---|
| | %C | *%H* | *%N* | *%Cl* |
| *Calculé*: | *53,50* | *3,88* | *14,18* | *7,18* |
| *Trouvé*: | *53,93* | *3,97* | *14, 06* | *7, 28* |

### EXEMPLE 13 : 3-{[2,2-Difluoro-2-(1-oxydo-2-pyridyl)-éthyl]-amino}-N-(2,3-difluorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2,3-difluorobenzylamine.

*Point de fusion : 142°C*

| *Microanalyse élémentaire :* | | | |
|---|---|---|---|
| | % *C* | *%H* | *%N* |
| *Calculé*: | *55,35* | *4, 01* | *14, 67* |
| *Trouvé*: | *55,34* | *4, 26* | *14,42* |

### EXEMPLE 14 : 3-{[2,2-Difluoro-2-(1-oxydo-2-pyridyl)-éthyl]-amino}-N-(3,5-difluorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 3,5-difluorobenzylamine.

*Point de fusion : 218-219°C*

| *Microanalyse élémentaire :* | | | |
|---|---|---|---|
| | *%C* | *%H* | *%N* |
| *Calculé*: | *55,35* | *4, 01* | *14, 67* |
| *Trouvé*: | *54,72* | *3,82* | *14,40* |

### EXEMPLE 15 : 3-{[2,2-Difluoro-2-(1-oxydo-2-pyridyl)-éthyl]-amino}-4-oxo-N-[2-(2-oxo-2-{[2-(2-pyridyl)-éthyl]-amino}-éthoxy)-benzyl]-4,6,7,8-tétrabydropyrrolo[1,2-a]pyrazine-6-carboxamide, chlorhydrate

### Stade A: 3-{[2,2-Difluoro-2-(1-oxydo-2-pyridyl)-éthyl]-amino}-4-oxo-N-[2-(2-oxo-2-{[2-(2 pyridyl)-éthyl]-amino}-éthoxy)-benzyl]-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par le 2-[2-(aminométhyl)-phénoxy]-N-[2-(2-pyridyl)-éthyl]-acétamide.

### Stade B: 3-{[2,2-Dijluoro-2-(1-oxydo-2-pyridyl)-éthyl]-amino}-4-oxo-N-[2-(2-oxo-2-{[2-(2-pyridyl)-éthyl]-amino}-éthoxy)-benzyl]-4,6,7,8-tétrahydropyrrolo[1,2-α]pyrazine-6-carboxamide, chlorhydrate

Le produit attendu est obtenu par acidification du composé obtenu au stade précédent par l'acide chlorhydrique.

| *Microanalyse élémentaire :* | | | | |
|---|---|---|---|---|
| | %C | *%H* | *%N* | *%Cl* |
| *Calculé*: | *56,75* | *4,92* | *14,94* | *5,40* |
| *Trouvé*: | *57, 22* | *4,85* | *14,87* | *5,82* |

### EXEMPLE 16 : (6S)-3-{[2,2-Difluoro-2-(1-oxydo-2-pyridyl)-éthyl]-amino}-N-(2,6-difluorobenzyl)-4-oxo-4,6,7,8-tétrahydropyrrolo[1,2-a]pyrazine-6-carboxamide

Le produit attendu est obtenu par séparation sur colonne HPLC chirale du mélange racémique de l'exemple 7.

*Pouvoir rotatoire : α_{D}* = *118,46° (méthanol, 20°C, c=0,95)*

### EXEMPLE 17 : (6S)-3-{[2,2-Difluoro-2-(1-oxydo-2-pyridyl)-éthyl]-amino}-N-(2,5-difluorobenzyl)4-oxo-4,6,7,8-tétrabydropyrrolo[1,2-a]pyrazine-6-carboxamide

Le produit attendu est obtenu par séparation sur colonne HPLC chirale du mélange racémique de l'exemple 10.

*Pouvoir rotatoire : α_{D} = - 89,65° (méthanol, 20°C, c=0,57)*

### EXEMPLE 18 : (6S)-3-{[2,2-Difluoro-2-(1-oxydo-2-pyridyl)-éthyl]-amino}-N-(2-chloro-6-fluorobenzyl)-4-oxo-4,6,7,8-tétrahydropyrrolo[1,2-a]pyrazine-6-carboxamide

Le produit attendu est obtenu par séparation sur colonne HPLC chirale du mélange racémique de l'exemple 12.

*Pouvoir rotatoire : α_{D} = - 101,49° (méthanol, 20°C, c=1,3)*

### EXEMPLE 19 : (6S)-3-{[2,2-Difluoro-2-(1-oxydo-2-pyridyl)-éthyl]-amino}-N-(2,3-difluorobenzyl)-4-oxo-4,6,7,8-tétrahydropyrrolo [1,2-a]pyrazine-6-carboxamide

Le produit attendu est obtenu par séparation sur colonne HPLC chirale du mélange racémique de l'exemple 13.

*Pouvoir rotatoire : α_{D} = - 102,06° (méthanol, 20°C, c=0,8)*

### EXEMPLE 20 : (6S)-3-{[2,2-Difluoro-2-(1-oxydo-2-pyridyl)-éthyl]-amino}-N-(2-chlorobenzyl)-4-oxo-4,6,7,8-tétrahydropyrrolo[1,2-a]pyrazine-6-carboxamide

Le produit attendu est obtenu par séparation sur colonne HPLC chirale du mélange racémique de l'exemple 8.

*Pouvoir rotatoire : α_{D} = - 105,65° (méthanol, 20°C*, *c=0,85)*

### EXEMPLE 21 : (6S)-3-{[2,2-Difluoro-2-(1-oxydo-2-pyridyl)-éthyl]-amino}-N-(2,3,6-trifluorobenzyl)-4-oxo-4,6,7,8-tétrabydropyrrolo[1,2-a]pyrazine-6-carboxamide

### Stade A: 3-{[2,2-Difluoro-2-(1-oxydo-2-pyridyl)-éthyl]-amino}-N-(2,3,6-trifluorobenzyl)-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 2,3,6-trifluorobenzylamine.

### Stade B: (6S)-3-{[2,2-Difluoro-2-(1-oxydo-2-pyridyl)-éthyl]-amino}-N-(2,3,6-trifluorobenzyl)-4-oxo-4,6,7,8-tétrahydropyrrolo[1,2-a]pyrazine-6-carboxamide

Le produit attendu est obtenu par séparation sur colonne HPLC chirale du mélange racémique obtenu au stade précédent.

*Pouvoir rotatoire : α_{D} 118*,*25° (méthanol, 20°C, c=1*)

### EXEMPLE 22 : (6S)-3-{[2,2-Difluoro-2-(1-oxydo-2-pyridyl)-éthyl]-amino}-N-benzyl-4-oxo-4,6,7,8-tétrahydropyrrolo[1,2-a]pyrazine-6-carboxamide, chlorhydrate

### Stade A: 3-{[2,2-Difluoro-2-(1-oxydo-2-pyridyl)-éthyl]-amino}-N-benzyl-4-oxo-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la benzylamine.

### Stade B: (6S)-3-{[2,2-Difluoro-2-(1-oxydo-2 pyridyl)-éthyl]-amino}-N-benzyl-4-oxo-4,6,7,8-tétrahydropyrrolo[1, 2-α]pyrazine-6-carboxamide

Le produit attendu est obtenu par séparation sur colonne HPLC chirale du mélange racémique obtenu au stade précédent.

### Stade C: (6S)-3-{[2,2-Difluoro-2-(1-oxydo-2-pyridyl)-éthyl]-amino}-N-benzyl-4-oxo-4,6,7,8-tétrahydropyrrolo[1,2-α]pyrazine-6-carboxamide, chlorhydrate

Le produit attendu est obtenu par acidification du composé obtenu au stade précédent par l'acide chlorhydrique.

| *Microanalyse élémentaire :* | | | | |
|---|---|---|---|---|
| | *%C* | *%H* | *%N* | *%Cl* |
| *Calculé*: | *55,29* | *4,64* | *14,65* | *7,42* |
| *Trouvé*: | *54,84* | *4,81* | *14,16* | *7,16* |

### EXEMPLE 23 : (6S)-3-{[2,2-Difluoro-2-(1-oxydo-2-pyridyl)-éthyl]-amino}-N-(3-thiénylméthyl)-4-oxo-4,6,7,8-tétrahydropyrrolo [1,2-a]pyrazine-6-carboxamide, chlorhydrate

Le produit attendu est obtenu selon le procédé décrit à l'exemple 22, en remplaçant au stade A la benzylamine par la 3-thiénylméthylamine.

| *Microanalyse élémentaire :* | | | | | |
|---|---|---|---|---|---|
| | *% C* | *% H* | *% N* | *%Cl* | %S |
| *Calculé*: | *49, 64* | *4,17* | *14,47* | *7,33* | *6, 63* |
| *Trouvé*: | *49, 09* | *4,17* | *14,14* | *8,16* | *6,59* |

### EXEMPLE 24 : (6S)-3-{[2,2-Difluoro-2-(1-oxydo-2-pyridyl)-éthyl]-amino}-N-(2-thiénylméthyl)-4-oxo-4,6,7,8-tétrahydropyrrolo[1,2-a]pyrazine-6-carboxamide, chlorhydrate

Le produit attendu est obtenu selon le procédé décrit à l'exemple 22, en remplaçant au stade A la benzylamine par la 2-thiénylméthylamine.

| *Microanalyse élémentaire :* | | | | | |
|---|---|---|---|---|---|
| | *% C* | *% H* | *% N* | *%Cl* | %S |
| *Calculé*: | *49, 64* | *4,17* | *14,47* | *7,33* | *6, 63* |
| *Trouvé*: | *50, 61* | *4,12* | *14,35* | *7,98* | *6, 65* |

### EXEMPLE 25 : (6S)-3-{[2,2-Difluoro-2-(1-oxydo-4-pyridyl)-éthyl]-amino}-N-(2-fluorobenzyl)-4-oxo-4,6,7,8-tétrahydropyrrolo[1,2-a]pyrazine-6-carboxamide

### Stade A: 3-{[2,2-Difluoro-2-(1-oxydo-4-pyridyl)-éthyl]-amino}-N-(2-fluorobenzyl)-4-oxo-4,6,7,8-tétrahydropyrrolo[1,2-α]pyrazine-6-carboxamide

Le produit attendu est obtenu selon le procédé décrit dans l'exemple 1, en remplaçant, au stade J, la 2,2-difluoro-2-(1-oxydo-2-pyridyl)-éthanamine par la 2,2-difluoro-2-(1-oxydo-4-pyridyl)-éthanamine.

### Stade B: (6S)-3-{[2,2-Difluoro-2-(1-oxydo-4-pyridyl)-éthyl]-amino}-N-(2-fluorobenzyl)-4-oxo-4, 6, 7, 8-tétrahydropyrrolo[1,2-α]pyrazine-6-carboxamide

Le produit attendu est obtenu par séparation sur colonne HPLC chirale du mélange racémique obtenu au stade précédent.

| *Microanalyse élémentaire :* | | | |
|---|---|---|---|
| | *% C* | *%H* | *%N* |
| *Calculé*: | *57,52* | *4,39* | *15,24* |
| *Trouvé*: | *57,73* | *4, 61* | *15, 05* |

### EXEMPLE 26 : (6S)-3-{[2,2-Difluoro-2-(1-oxydo-3-pyridyl)-éthyl]-amino}-N-(2-fluorobenzyl)-4-oxo-4,b,7,8-tétrahydropyrrolo[1,2-a]pyrazine-6-carboxamide

Le produit attendu est obtenu selon le procédé de l'exemple 25, en remplaçant au stade A la 2,2-difluoro-2-(1-oxydo-4-pyridyl)-éthanamine par la 2,2-difluoro-2-(1-oxydo-3-pyridyl)-éthanamine.

| *Microanalyse élémentaire :* | | | |
|---|---|---|---|
| | *%C* | *%H* | *%N* |
| *Calculé*: | *57,52* | *4,39* | *15,24* |
| *Trouvé*: | *57,34* | *4,49* | *14,99* |

### EXEMPLE 27 : 3-{[2,2-Difluoro-2-(1-oxydo-2-pyridyl)-éthyl]-amino}-8,8-diméthyl-N-(2-fluorobenzyl)-4-oxo-4,6,7,8-tétrahydropyrrolo[1,2-a]pyrazine-6-carboxamide

Le produit attendu est obtenu selon le procédé de l'exemple 1, en remplaçant au stade A le 5-oxoprolinate de benzyle par le 4,4-diméthyl-5-oxo-2-pyrrolidinecarboxylate de benzyle.

### EXEMPLE 28 : (6R)-3-{[2,2-Difluoro-2-(1-oxydo-2-pyridyl)-éthyl]-amino}-8,8-diméthyl-N-(2-fluorobenzyl)-4-oxo-4,6,7,8-tétrahydropyrrolo[1,2-a]pyrazine-6-carboxamide

Le mélange racémique de l'exemple 27 est séparé par chromatographie HPLC préparative sur phase chirale.
Le produit attendu est le premier des énantiomères ainsi obtenu.

| *Microanalyse élémentaire :* | | | |
|---|---|---|---|
| | *% C* | *%H* | *%N* |
| *Calculé*: | *59,13* | *4,96* | *14,37* |
| *Trouvé*: | *58, 88* | *4, 99* | *14, 08* |

### EXEMPLE 29 : (6S)-3-{[2,2-Ditluoro-2-(1-oxydo-2-pyridyl)-éthyl]-amino}-8,8-diméthyl-N-(2-fluorobenzyl)-4-oxo-4,6,7,8-tétrahydropyrrolo[1,2-a]pyrazine-6-carboxamide

Le mélange racémique de l'exemple 27 est séparé par chromatographie HPLC préparative sur phase chirale.
Le produit attendu est le deuxième des énantiomères ainsi obtenu.

| *Microanalyse élémentaire :* | | | |
|---|---|---|---|
| | *% C* | *%H* | *%N* |
| *Calculé*: | *59,13* | *4,96* | *14,37* |
| *Trouvé*: | *58,92* | *4,93* | *14,14* |

### EXEMPLE 30 : (6S)-3-{[2,2-Difluoro-2-(1-oxydo-2-pyridyl)-éthyl]-amino}-4-oxo-N-[(5-oxo-4,5-dihydro-1H-1,2,4-triazol-3-yl)-méthyl]-4,6,7,8-tétrahydropyrrolo[1,2-a]pyrazine-6-carboxamide

### Stade A: 3-{[2,2-Difluoro-2-(1-oxydo-2-pyridyl)-éthyl]-amino}-4-oxo-N-[(5-oxo-4,5-dihydro-1H-1,2,4-triazol-3-yl)-méthyl]-4,6,7,8-tétrahydropyrrolo[1,2-a]pyrazine-6-carboxamide

Le produit attendu est obtenu selon le procédé décrit dans l'exemple 1, en remplaçant, au stade L, la 2-fluorobenzylamine par la 5-(aminométhyl)-2,4-dihydro-3*H*-1,2,4-triazol-3-one.

### Stade B: (6S)-3-{[2,2-Difluoro-2-(1-oxydo-2-pyridyl)-éthyl]-amino}-4-oxo-N-[(5-oxo-4,5-dihydro-1H-1,2,4-triazol-3 yl)-méthyl]-4,6,7,8-tétrahydropyrrolo[1,2-a]pyrazine-6-carboxamide

Le produit attendu est obtenu par séparation sur colonne HPLC chirale du mélange racémique obtenu au stade précédent.

| *Microanalyse élémentaire :* | | | |
|---|---|---|---|
| | *%C* | *%H* | *%N* |
| *Calculé*: | *48*,*22* | *4*,*05* | *24*,*99* |
| *Trouvé*: | *48*,*61* | *4*,*34* | *24*,*56* |

### EXEMPLE 31 : (6S)-3-{[2,2-Difluoro-2-(1-oxydo-2-quinoléinyl)-éthyl]-amino}-N-(2-fluorobenzyl)-4-oxo-4,6,7,8-tétrahydropyrrolo[1,2-a]pyrazine-6-carboxamide

### Stade A: 3-{[2,2-Difluoro-2-(1-oxydo-2-quinoléinyl)-éthyl]-amino}-N-(2-fluorobenzyl)-4-oxo-4,6,7,8-tétrahydropyrrolo[1,2-α]pyrazine-6-carboxamide

Le produit attendu est obtenu selon le procédé décrit dans l'exemple 1, en remplaçant, au stade J, la 2,2-difluoro-2-(1-oxydo-2-pyridyl)-éthanamine par la 2,2-difluoro-2-(1-oxydo-2-quinoléinyl)-éthanamine.

### Stade B: (6S)-3-{[2,2-Difluoro-2-(1-oxydo-2-quinoléinyl)-éthyl]-amino}-N-(2-fluorobenzyl)-4-oxo-4,6,7,8-tétrahydropyrrolo[1,2-α]pyrazine-6-carboxamide

Le produit attendu est obtenu par séparation sur colonne HPLC chirale du mélange racémique obtenu au stade précédent.

| *Microanalyse élémentaire :* | | | |
|---|---|---|---|
| | *% C* | *%H* | *%N* |
| *Calculé*: | *61,29* | *4,35* | *13,75* |
| *Trouvé*: | *61, 65* | *4, 51* | *13,50* |

### ETUDE PHARMACOLOGIOUE DES DERIVES DE L'INVENTION

### EXEMPLE 32 : Inhibition de la thrombine et de protéases à sérine de la fibrinolyse

Pour évaluer in vitro l'activité inhibitrice des produits de l'invention sur la thrombine humaine (Sigma, activité spécifique 3230 UNIH/mg), le fibrinogène humain purifié (4 mM, Stago) (Fg) a été ajouté à une quantité donnée de thrombine (0.7 nM) préalablement incubée avec ou sans l'inhibiteur à tester (20°C, 10 minutes).

Inhibiteurs, enzymes et substrats sont dilués dans le même tampon (tampon phosphate 0.01 mM, pH 7.4, contenant 0.12 M de chlorure de sodium et 0.05 % de sérum albumine bovine) puis distribués dans une microplaque en polystyrène sous un volume de 50 µl.

La fibrine formée par la thrombine est mesurée spectrophotométriquement à 405 nm après 10 à 15 minutes de réaction à 20°C.

Le tableau ci-dessous donne la concentration des composés en nM inhibant 50 % de l'activité enzymatique (CI₅₀) de la thrombine par rapport au contrôle sans produit. Les résultats obtenus démontrent que les composés de l'invention sont des inhibiteurs puissants de la thrombine humaine vis-à-vis du fibrinogène humain.

**Tableau**

| *Exemple* | *CI₅₀ (nM)* |
|---|---|
| 1 | 28 |
| 3 | 16 |
| 8 | 38 |
| 15 | 1,4 |
| 21 | 11 |
| 24 | 60 |
| 31 | 8,4 |

### EXEMPLE 33 : Composition pharmaceutique

| Formule de préparation pour 1000 comprimés dosés à 10 mg : | |
|---|---|
| Composé de l'exemple 3 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 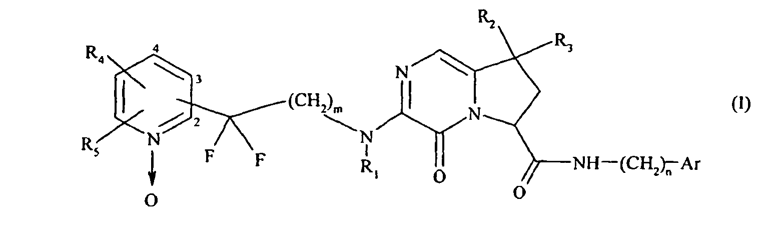 dans laquelle :
* 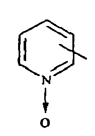 représente un groupement 1-oxydo-pyridinyle substitué par le reste de la molécule en l'une quelconque des positions 2, 3 ou 4,
* m et n, identiques ou différents, représentent chacun un entier compris entre 1 et 3,
* R₁ représente un atome d'hydrogène ou un groupement alkyle (C₁-C₆) linéaire ou ramifié,
* R₂ et R₃, identiques ou différents, représentent chacun un atome ou groupement choisi parmi les atomes d'hydrogène et d'halogène et les groupements alkyle (C₁-C₆) linéaire ou ramifié, hydroxy, acyloxy (C₁-C₆) linéaire ou ramifié et alkoxy (C₁-C₆) linéaire ou ramifié, ou forment ensemble, avec l'atome de carbone qui les porte, un cycloalcane ayant de 3 à 6 atomes de carbone,
* R₄ et R₅ représentent chacun un atome d'hydrogène, ou sont adjacents et forment ensemble, avec les atomes de carbone qui les portent, un cycle benzo,
* Ar représente un groupement aryle ou hétéroaryle,
ses énantiomères, ainsi que ses sels d'addition à un acide pharmaceutiquement acceptable,
étant entendu que
par groupement aryle , on entend phényle, biphénylyle ou naphtyle, chacun de ces groupements étant éventuellement substitué par un ou plusieurs groupements, identiques ou différents, choisis parmi :
- halogène,
- alkyle (C₁-C₆) linéaire ou ramifié éventuellement substitué par un groupement hydroxy, carboxy ou carbamoyle, le groupement carbamoyle étant lui-même éventuellement substitué par un ou deux groupements alkyle (C₁-C₆) linéaire ou ramifié,
- alkoxy (C₁-C₆) linéaire ou ramifié,
- hydroxy,
- trihalogénoalkyle (C₁-C₆) linéaire ou ramifié,
- amino éventuellement substitué par un ou deux groupements alkyle (C₁-C₆) linéaire ou ramifié,
- carboxyméthoxy,
- et carbamoylméthoxy éventuellement N-substitué par un ou deux groupements choisis parmi alkyle (C₁-C₆) linéaire ou ramifié, hydroxyalkyle (C₁-C₆) linéaire ou ramifié, alkoxyalkyle où les parties alkoxy et alkyle sont chacune en (C₁-C₆) linéaire ou ramifié, et pyridylalkyle où la partie alkyle est en (C₁-C₆) linéaire ou ramifié,
et par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi :
- halogène,
- alkyle (C₁-C₆) linéaire ou ramifié éventuellement substitué par un groupement hydroxy, carboxy ou carbamoyle, le groupement carbamoyle étant lui-même éventuellement substitué par un ou deux groupements alkyle (C₁-C₆) linéaire ou ramifié,
- hydroxy,
- oxo,
- alkoxy (C₁-C₆) linéaire ou ramifié,
- trihalogénoalkyle (C₁-C₆) linéaire ou ramifié,
- amino éventuellement N-substitué par un ou deux groupements alkyle (C₁-C₆) linéaire ou ramifié,
- carboxyméthoxy,
- et carbamoylméthoxy éventuellement N-substitué par un ou deux groupements choisis parmi alkyle (C₁-C₆) linéaire ou ramifié, hydroxyalkyle (C₁-C₆) linéaire ou ramifié, alkoxyalkyle où les parties alkoxy et alkyle sont chacune en (C₁-C₆) linéaire ou ramifié, et pyridylalkyle où la partie alkyle est en (C₁-C₆) linéaire ou ramifié.

2. Composé de formule (I) selon la revendication 1, pour lequel la configuration du centre asymétrique en alpha de l'amide est (S).

3. Composé de formule (I) selon l'une quelconque des revendications 1 ou 2, pour lequel m est égal à 1, ses énantiomères, ainsi que ses sels d'addition à un acide pharmaceutiquement acceptable.

4. Composé de formule (I) selon l'une quelconque des revendications 1 à 3, pour lequel n est égal à 1, ses énantiomères, ainsi que ses sels d'addition à un acide pharmaceutiquement acceptable.

5. Composé de formule (I) selon l'une quelconque des revendications 1 à 4, pour lequel R₁ représente un atome d'hydrogène, ses énantiomères, ainsi que ses sels d'addition à un acide pharmaceutiquement acceptable.

6. Composé de formule (I) selon l'une quelconque des revendications 1 à 5, pour lequel R₂ représente un atome d'hydrogène, ses énantiomères, ainsi que ses sels d'addition à un acide pharmaceutiquement acceptable.

7. Composé de formule (I) selon l'une quelconque des revendications 1 à 6, pour lequel R₃ représente un atome d'hydrogène, ses énantiomères, ainsi que ses sels d'addition à un acide pharmaceutiquement acceptable.

8. Composé de formule (I) selon l'une quelconque des revendications 1 à 7, pour lequel R₄ et R₅ représentent chacun un atome d'hydrogène, ses énantiomères, ainsi que ses sels d'addition à un acide pharmaceutiquement acceptable.

9. Composé de formule (I) selon l'une quelconque des revendications 1 à 7, pour lequel R₄ et R₅ sont adjacents et forment ensemble, avec les atomes de carbone qui les portent, un cycle benzo, ses énantiomères, ainsi que ses sels d'addition à un acide pharmaceutiquement acceptable.

10. Composé de formule (I) selon l'une quelconque des revendications 1 à 9 pour lequel Ar représente un groupement phényle, thiényle ou pyridyle, chacun de ces groupements étant non substitué ou substitué par un ou plusieurs groupements, identiques ou différents, choisis parmi :
- halogène,
- alkyle (C₁-C₆) linéaire ou ramifié éventuellement substitué par un groupement hydroxy, carboxy ou carbamoyle, le groupement carbamoyle étant lui-même éventuellement substitué par un ou deux groupements alkyle (C₁-C₆) linéaire ou ramifié,
- alkoxy (C₁-C₆) linéaire ou ramifié,
- hydroxy,
- trihalogénoalkyle (C₁-C₆) linéaire ou ramifié,
- amino éventuellement substitué par un ou deux groupements alkyle (C₁-C₆) linéaire ou ramifié,
- carboxyméthoxy,
- et carbamoylméthoxy éventuellement N-substitué par un ou deux groupements choisis parmi alkyle (C₁-C₆) linéaire ou ramifié, hydroxyalkyle (C₁-C₆) linéaire ou ramifié, alkoxyalkyle où les parties alkoxy et alkyle sont chacune en (C₁-C₆) linéaire ou ramifié, et pyridylalkyle où la partie alkyle est en (C₁-C₆) linéaire ou ramifié,
ses énantiomères, ainsi que ses sels d'addition à un acide pharmaceutiquement acceptable.

11. Composé de formule (I) selon la revendication 10, pour lequel Ar représente un groupement phényle non substitué ou substitué par un ou plusieurs atomes d'halogène, identiques ou différents, choisis parmi fluor et chlore, ses énantiomères, ainsi que ses sels d'addition à un acide pharmaceutiquement acceptable.

12. Composé de formule (I) selon la revendication 1 choisi parmi :
- le 3-{[2,2-difluoro-2-(1-oxydo-2-pyridyl)-éthyl]-amino}-N-(2-fluorobenzyl)-4-oxo-4,6, 7,8-tétrahydropyrrolo[1,2-a]pyrazine-6-carboxamide, ainsi que son énantiomère (6S),
- le 3-{[2,2-difluoro-2-(1-oxydo-2-pyridyl)-éthyl]-amino}-N-(2,6-difluorobenzyl)-4-oxo-4,6,7,8-tétrahydropyrrolo[1,2-a]pyrazine-6-carboxamide, ainsi que son énantiomère (6S),
- le 3-{[2,2-difluoro-2-(1-oxydo-2-pyridyl)-éthyl]-amino}-N-(2-chlorobenzyl)-4-oxo-4, 6,7,8-tétrahydropyrrolo[1,2-a]pyrazine-6-carboxamide, ainsi que son énantiomère (6S) ;
- le 3-{[2,2-difluoro-2-(1-oxydo-2-pyridyl)-éthyl]-amino}-N-(2,5-difluorobenzyl)-4-oxo-4,6,7,8-tétrahydropyrrolo[1,2-a]pyrazine-6-carboxamide, ainsi que son énantiomère (6S),
- le 3-{[2,2-difluoro-2-(1-oxydo-2-pyridyl)-éthyl]-amino}-N-(2,3-difluorobenzyl)-4-oxo-4,6,7,8-tétrahydropyrrolo[1,2-a]pyrazine-6-carboxamide, ainsi que son énantiomère (6S),
- et le 3-{[2,2-difluoro-2-(1-oxydo-2-pyridyl)-éthyl]-amino}-N-(2,3,6-trifluorobenzyl)-4-oxo-4,6,7,8-tétrahydropyrrolo[1,2-a]pyrazine-6-carboxamide, ainsi que son énantiomère (6S).

13. Procédé de préparation des composés de formule (I) selon la revendication 1, dans lequel on réduit un composé de formule (II) : 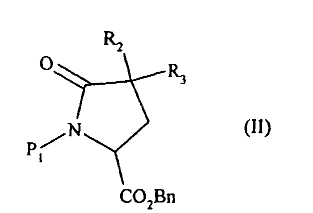 dans laquelle R₂ et R₃ sont tels que définis dans la formule (I), P₁ représente un groupement protecteur de la fonction amino et Bn représente le groupement benzyle,
à l'aide d'un agent réducteur,
pour conduire au composé de formule (III) : 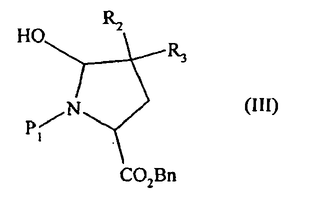 dans laquelle R₂, R₃, P₁ et Bn ont la même signification que précédemment,
dont on transforme la fonction hydroxy en méthoxy puis en fonction cyano par des réactions classiques de la chimie organique, pour conduire, après déprotection de la fonction amino, au composé de formule (IV): 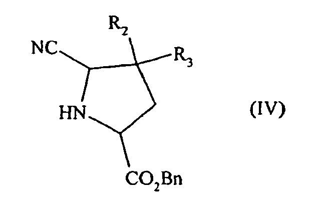 dans laquelle R₂, R₃, et Bn ont la même signification que précédemment,
que l'on met en réaction avec du chlorure d'oxalyle pour conduire au composé de formule (V) : 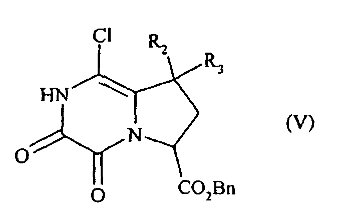 dans laquelle R₂, R₃ et Bn ont la même signification que précédemment,
que l'on soumet à une réaction d'hydrogénation catalytique, pour conduire au composé de formule (VI) : 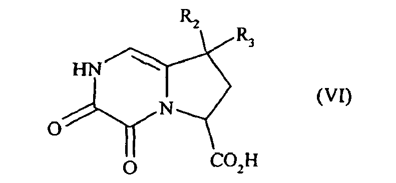 dans laquelle R₂ et R₃ sont tels que définis précédemment,
que l'on estérifie en composé de formule (VII) : 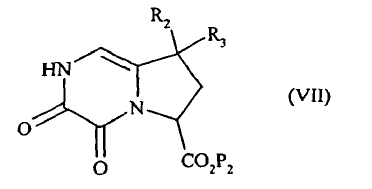 dans laquelle R₂ et R₃ sont tels que définis précédemment, et P₂ représente un groupement alkyle (C₁-C₆) linéaire ou ramifié,
que l'on met en réaction avec un agent de bromation, pour conduire au composé de formule (VIII) : 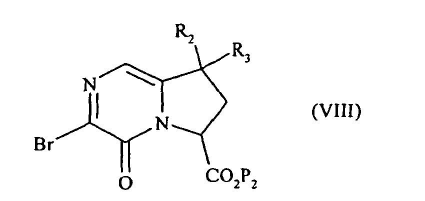 dans laquelle R₂, R₃ et P₂ sont tels que définis précédemment,
que l'on met en réaction avec la 2-mercaptopyridine, pour conduire au composé de formule (IX) : 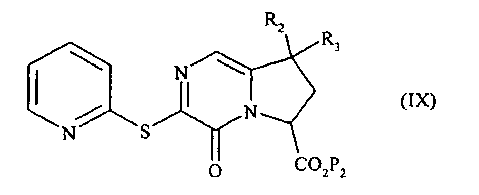 dans laquelle R₂, R₃ et P₂ sont tels que définis précédemment,
que l'on met en réaction avec le N-oxyde de formule (X) : 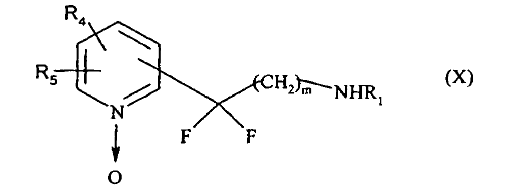 dans laquelle m, R₁, R₄ et R₅ sont tels que définis dans la formule (I),
pour conduire au composé de formule (XI) : 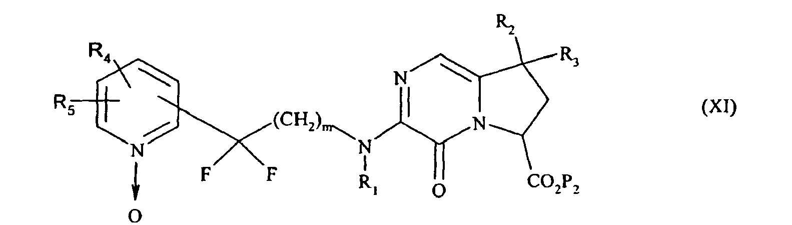 dans laquelle m, R₁, R₂, R₃ R₄, R₅ et P₂ sont tels que définis précédemment,
dont on déprotège la fonction acide, pour conduire au composé de formule (XII) : 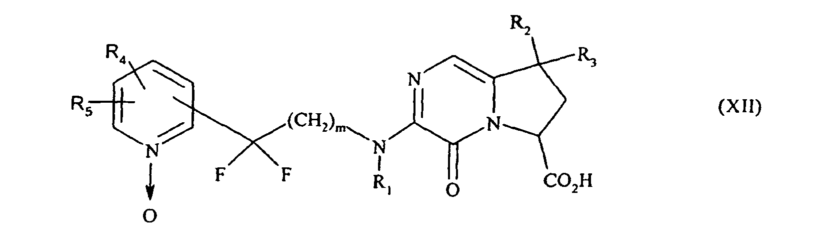 dans laquelle m, R₁, R₂, R₃ R₄ et R₅ sont tels que définis précédemment,
que l'on met en réaction avec un composé de formule (XIII) : 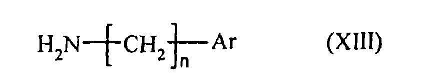 dans laquelle n et Ar ont la même signification que dans la formule (I),
en présence d'un agent de couplage,
pour conduire au composé de formule (I).

14. Composition pharmaceutique contenant comme principe actif un composé selon l'une quelconque des revendications 1 à 12, en combinaison avec un ou plusieurs véhicules inertes, non toxiques et pharmaceutiquement acceptables.

15. Utilisation des composés de formule (I) selon l'une quelconque des revendications 1 à 12 pour la fabrication de médicaments utiles en tant qu'inhibiteurs de thrombine.

16. Utilisation des composés de formule (I) selon l'une quelconque des revendications 1 à 12 pour la fabrication de médicaments utiles dans le traitement des angines stables ou non, des maladies d'origine thrombotique et/ou donnant lieu à des complications thrombotiques, dans le traitement ou la prévention de l'infarctus du myocarde et des thromboses veineuses ou artérielles, ainsi que dans le traitement des complications des maladies vasculaires et cardiovasculaires telles que l'athérosclérose, l'artérite, la maladie veineuse, et dans le traitement de toutes les maladies impliquant une formation et/ou une activité de la thrombine.

## Claims

1. Compound of formula (I) : 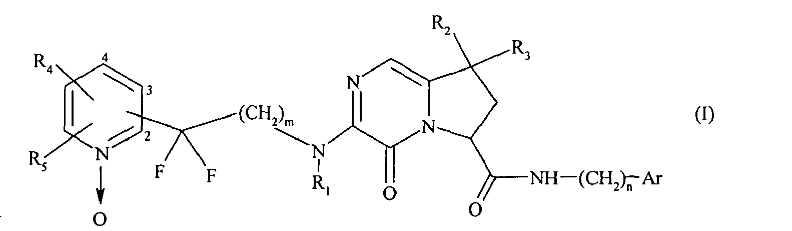 wherein :
* 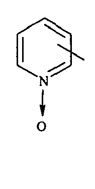 represents a 1-oxidopyridyl group substituted by the remainder of the molecule in any one of the positions 2, 3 and 4,
* m and n, which may be identical or different, each represent an integer of from 1 to 3,
* R₁ represents a hydrogen atom or a linear or branched (C₁-C₆)alkyl group,
* R₂ and R₃, which may be identical or different, each represent an atom or group selected from the atoms hydrogen and halogen and the groups linear or branched (C₁-C₆)alkyl, hydroxy, linear or branched (C₁-C₆)acyloxy and linear or branched (C₁-C₆)alkoxy, or together form, with the carbon atom carrying them, a cycloalkane having from 3 to 6 carbon atoms,
* R₄ and R₅ each represent a hydrogen atom, or are adjacent and together form, with the carbon atoms carrying them, a benzo ring,
* Ar represents an aryl or heteroaryl group,
its enantiomers, and addition salts thereof with a pharmaceutically acceptable acid,
"aryl group" being understood to mean phenyl, biphenylyl or naphthyl, each of those groups being optionally substituted by one or more identical or different groups selected from :
- halogen,
- linear or branched (C₁-C₆)alkyl optionally substituted by a hydroxy, carboxy or carbamoyl group, the carbamoyl group being itself optionally substituted by one or two linear or branched (C₁-C₆)alkyl groups,
- linear or branched (C₁-C₆)alkoxy,
- hydroxy,
- trihalo-(C₁-C₆)alkyl in which the alkyl moiety may be linear or branched,
- amino optionally substituted by one or two linear or branched (C₁-C₆)alkyl groups,
- carboxymethoxy,
- and carbamoylmethoxy optionally N-substituted by one or two groups selected from linear or branched (C₁-C₆)alkyl, hydroxy-(C₁-C₆)alkyl in which the alkyl moiety may be linear or branched, alkoxyalkyl in which the alkoxy and alkyl moieties are each linear or branched C₁-C₆, and pyridylalkyl in which the alkyl moiety is linear or branched C₁-C₆,
and "heteroaryl group" being understood to mean a mono- or bi-cyclic aromatic group having from 5 to 12 ring members and containing one, two or three hetero atoms selected from oxygen, nitrogen and sulphur, it being understood that the heteroaryl may be optionally substituted by one or more identical or different groups selected from :
- halogen,
- linear or branched (C₁-C₆)alkyl optionally substituted by a hydroxy, carboxy or carbamoyl group, the carbamoyl group being itself optionally substituted by one or two linear or branched (C₁-C₆)alkyl groups,
- hydroxy,
- oxo,
- linear or branched (C₁-C₆)alkoxy,
- trihalo-(C₁-C₆)alkyl in which the alkyl moiety may be linear or branched,
- amino optionally N-substituted by one or two linear or branched (C₁-C₆)alkyl groups,
- carboxymethoxy,
- and carbamoylmethoxy optionally N-substituted by one or two groups selected from linear or branched (C₁-C₆)alkyl, hydroxy-(C₁-C₆)alkyl in which the alkyl moiety may be linear or branched, alkoxyalkyl in which the alkoxy and alkyl moieties are each linear or branched C₁-C₆, and pyridylalkyl in which the alkyl moiety is linear or branched C₁-C₆.

2. Compound of formula (I) according to claim 1, wherein the configuration of the asymmetric centre at the alpha position with respect to the amide is (S).

3. Compound of formula (I) according to claim 1 or 2, wherein m is 1, its enantiomers, and its addition salts with a pharmaceutically acceptable acid.

4. Compound of formula (I) according to any one of claims 1 to 3, wherein n is 1, its enantiomers, and its addition salts with a pharmaceutically acceptable acid.

5. Compound of formula (I) according to any one of claims 1 to 4, wherein R₁ represents a hydrogen atom, its enantiomers, and its addition salts with a pharmaceutically acceptable acid.

6. Compound of formula (I) according to any one of claims 1 to 5, wherein R₂ represents a hydrogen atom, its enantiomers, and its addition salts with a pharmaceutically acceptable acid.

7. Compound of formula (I) according to any one of claims 1 to 6, wherein R₃ represents a hydrogen atom, its enantiomers, and its addition salts with a pharmaceutically acceptable acid.

8. Compound of formula (I) according to any one of claims 1 to 7, wherein R₄ and R₅ each represent a hydrogen atom, its enantiomers, and its addition salts with a pharmaceutically acceptable acid.

9. Compound of formula (I) according to any one of claims 1 to 7, wherein R₄ and R₅ are adjacent and together form, with the carbon atoms carrying them, a benzo ring, its enantiomers, and its addition salts with a pharmaceutically acceptable acid.

10. Compound of formula (I) according to any one of claims 1 to 9 wherein Ar represents a phenyl, thienyl or pyridyl group, each of those groups being unsubstituted or substituted by one or more identical or different groups selected from :
- halogen,
- linear or branched (C₁-C₆)alkyl optionally substituted by a hydroxy, carboxy or carbamoyl group, the carbamoyl group being itself optionally substituted by one or two linear or branched (C₁-C₆) alkyl groups,
- linear or branched (C₁-C₆) alkoxy,
- hydroxy,
- trihalo-(C₁-C₆)alkyl in which the alkyl moiety may be linear or branched,
- amino optionally substituted by one or two linear or branched (C₁-C₆)alkyl groups,
- carboxymethoxy,
- and carbamoylmethoxy optionally N-substituted by one or two groups selected from linear or branched (C₁-C₆)alkyl, hydroxy-(C₁-C₆)alkyl in which the alkyl moiety may be linear or branched, alkoxyalkyl in which the alkoxy and alkyl moieties are each linear or branched C₁-C₆, and pyridylalkyl in which the alkyl moiety is linear or branched C₁-C₆,
its enantiomers, and its addition salts with a pharmaceutically acceptable acid.

11. Compound of formula (I) according to claim 10, wherein Ar represents a phenyl group unsubstituted or substituted by one or more identical or different halogen atoms selected from fluorine and chlorine, its enantiomers, and its addition salts with a pharmaceutically acceptable acid.

12. Compound of formula (I) according to claim 1 selected from :
- 3-{[2,2-difluoro-2-(1-oxido-2-pyridyl)ethyl]amino}-N-(2-fluorobenzyl)-4-oxo-4,6,7,8-tetrahydropyrrolo[1,2-a]pyrazine-6-carboxamide, and its (6S) enantiomer,
- 3-{[2,2-difluoro-2-(1-oxido-2-pyridyl)ethyl]amino}-N-(2,6-difluorobenzyl)-4-oxo-4,6,7,8-tetrahydropyrrolo[1,2-a]pyrazine-6-carboxamide, and its (6S) enantiomer,
- 3-{[2,2-difluoro-2-(1-oxido-2-pyridyl)ethyl]amino}-N-(2-chlorobenzyl)-4-oxo-4,6,7,8-tetrahydropyrrolo[1,2-a]pyrazine-6-carboxamide, and its (6S) enantiomer ;
- 3-{[2,2-difluoro-2-(1-oxido-2-pyridyl)ethyl]amino}-N-(2,5-difluorobenzyl)-4-oxo-4,6,7,8-tetrahydropyrrolo[1,2-a]pyrazine-6-carboxamide, and its (6S) enantiomer,
- 3-{[2,2-difluoro-2-(1-oxido-2-pyridyl)ethyl]amino}-N-(2,3-difluorobenzyl)-4-oxo-4,6,7,8-tetrahydropyrrolo[1,2-a]pyrazine-6-carboxamide, and its (6S) enantiomer,
- and 3-{[2,2-difluoro-2-(1-oxido-2-pyridyl)ethyl]amino}-N-(2,3,6-trifluorobenzyl)-4-oxo-4,6,7,8-tetrahydropyrrolo[1,2-a]pyrazine-6-carboxamide, and its (6S) enantiomer.

13. Process for the preparation of compounds of formula (I) according to claim 1, wherein a compound of formula (II) : 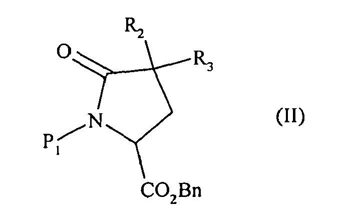 wherein R₂ and R₃ are as defined for formula (I), P₁ represents a protecting group for the amino function and Bn represents a benzyl group is reduced,
using a reducing agent,
to yield a compound of formula (III) : 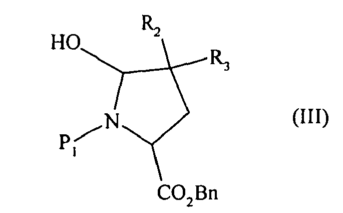 wherein R₂, R₃, P₁ and Bn are as defined hereinbefore,
the hydroxy function of which is converted to methoxy and then to the cyano function by conventional reactions of organic chemistry to yield, after deprotection of the amino function, a compound of formula (IV) : 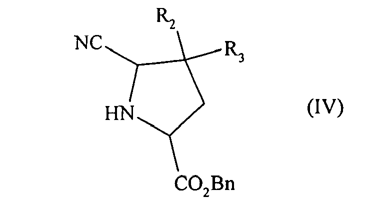 wherein R₂, R₃ and Bn are as defined hereinbefore,
which is reacted with oxalyl chloride to yield a compound of formula (V) : 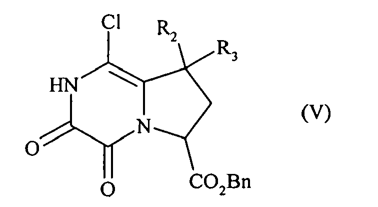 wherein R₂, R₃ and Bn are as defined hereinbefore,
which is subjected to catalytic hydrogenation to yield a compound of formula (VI) : 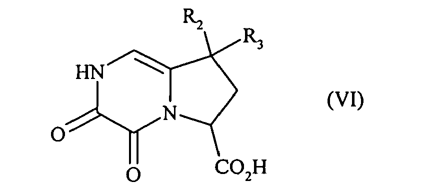 wherein R₂ and R₃ are as defined hereinbefore,
which is esterified to form a compound of formula (VII) : 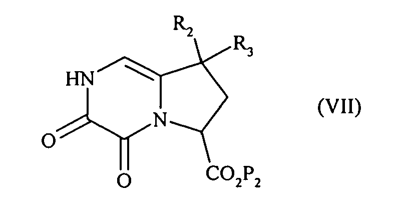 wherein R₂ and R₃ are as defined hereinbefore and P₂ represents a linear or branched (C₁-C₆)alkyl group,
which is reacted with a brominating agent to yield a compound of formula (VIII) : 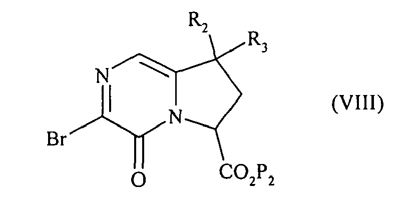 wherein R₂, R₃ and P₂ are as defined hereinbefore,
which is reacted with 2-mercaptopyridine to yield a compound of formula (IX) : 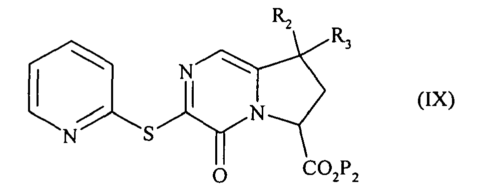 wherein R₂, R₃ and P₂ are as defined hereinbefore,
which is reacted with an N-oxide of formula (X) : 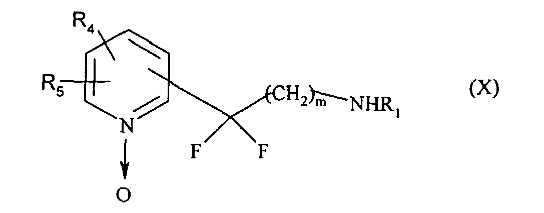 wherein m, R₁, R₄ and R₅ are as defined for formula (I),
to yield a compound of formula (XI) : 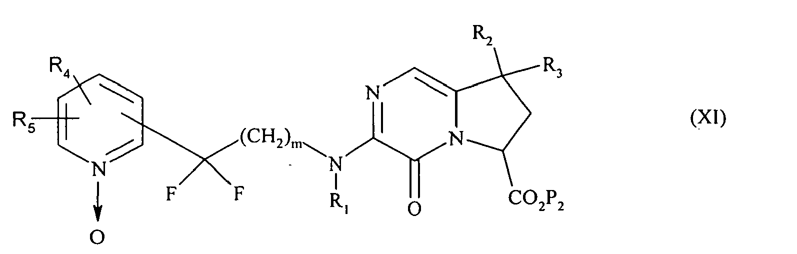 wherein m, R₁, R₂, R₃, R₄, R₅ and P₂ are as defined hereinbefore,
the acid function of which is deprotected to yield a compound of formula (XII) : 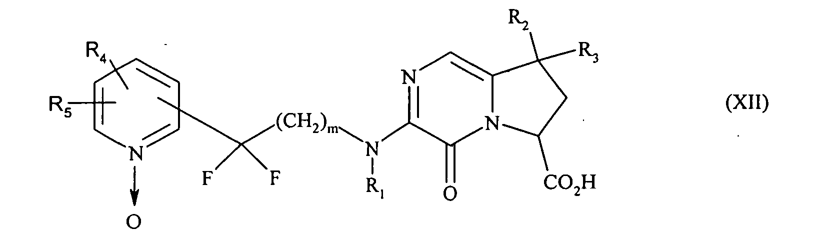 wherein m, R₁, R₂, R₃, R₄ and R₅ are as defined hereinbefore,
which is reacted with a compound of formula (XIII) : 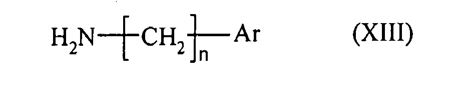 wherein n and Ar are as defined for formula (I),
in the presence of a coupling agent,
to yield a compound of formula (I).

14. Pharmaceutical composition containing as active ingredient a compound according to any one of claims 1 to 12 in combination with one or more pharmaceutically acceptable, inert, non-toxic carriers.

15. Use of the compounds of formula (I) according to any one of claims 1 to 12 for the manufacture of medicaments for use as thrombin inhibitors.

16. Use of the compounds of formula (I) according any one of claims 1 to 12 for the manufacture of medicaments for use in the treatment of stable and unstable angina, disorders of thrombotic origin and/or giving rise to thrombotic complications, in the treatment or prevention of myocardial infarction and venous or arterial thromboses, and in the treatment of complications of vascular and cardiovascular diseases such as atherosclerosis, arteritis, venous disease, and in the treatment of any disorders involving thrombin formation and/or activity.

## Patentansprüche

1. Verbindung der Formel (I): 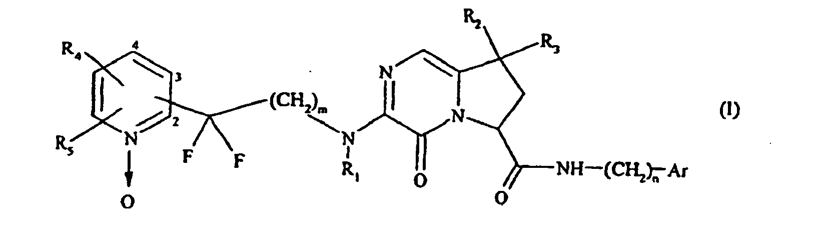 in der:
* 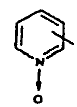 eine 1-Oxido-pyridinylgruppe bedeutet, die an irgendeiner der Positionen 2, 3 oder 4 mit dem Rest des Moleküls substituiert ist,
* m und n, die gleichartig oder verschieden sind, jeweils eine ganze Zahl mit einem Wert zwischen 1 und 3 bedeuten.
* R₁ ein Wasserstoffatom oder eine geradkettige oder verzweigte (C₁-C₆)-Alkylgruppe bedeutet,
* R₂ und R₃, die gleichartig oder verschieden sind, jeweils ein Atom oder eine Gruppe bedeuten ausgewählt aus Wasserstoff- und Halogenatomen und geradkettigen oder verzweigten (C₁-C₆)-Alkylgruppen, Hydroxygruppen, geradkettigen oder verzweigten (C₁-C₆)-Acyloxygruppen und geradkettigen oder verzweigten (C₁-C₆)-Alkoxygruppen, oder gemeinsam mit dem sie tragenden Kohlenstoffatom ein Cycloalkan mit 3 bis 6 Kohlenstoffatomen bilden,
* R₄ und R₅ jeweils ein Wasserstoffatom bedeuten oder benachbart sind und gemeinsam mit den sie tragenden Kohlenstoffatomen einen Benzoring bilden,
* Ar eine Aryl- oder Heteroarylgruppe bedeutet,
deren Enantiomere sowie deren Additionssalze mit einer pharmazeutisch annehmbaren Säure,
mit der Maßgabe, daß man
unter einer Arylgruppe ein Phenyl, Biphenyl oder Naphthyl versteht, wobei jede dieser Gruppen gegebenenfalls durch eine oder mehrere, gleichartige oder verschiedenartige Gruppen substituiert ist, ausgewählt aus:
- Halogen,
- geradkettigem oder verzweigtem (C₁-C₆)-Alkyl, welches gegebenenfalls durch eine Hydroxygruppe, Carboxygruppe oder Carbamoylgruppe substituiert ist, wobei die Carbamoylgruppe ihrerseits gegebenenfalls durch eine oder zwei geradkettige oder verzweigte (C₁-C₆)-Alkylgruppen substituiert ist,
- geradkettigem oder verzweigtem (C₁-C₆)-Alkoxy,
- Hydroxy,
- geradkettigem oder verzweigtem (C₁-C₆)-Trihalogenalkyl,
- Amino, gegebenenfalls substituiert durch eine oder zwei geradkettige oder verzweigte (C₁-C₆)-Alkylgruppen.
- Carboxymethoxy
- und Carbamoylmethoxy, gegebenenfalls N-substituiert durch eine oder zwei Gruppen ausgewählt aus geradkettigem oder verzweigtem (C₁-C₆)-Alkyl, geradkettigem oder verzweigtem (C₁-C₆)-Hydroxyalkyl, Alkoxyalkyl, wobei die Alkoxy- und Alkylreste jeweils geradkettige oder verzweigte (C₁-C₆)-Gruppen sind, und der Alkylrest der Pyridylalkylgruppe eine geradkettige oder verzweigte (C₁-C₆)-Gruppe ist,
und man unter einer Heteroarylgruppe eine mono- oder bicyclische aromatische Gruppe mit 5 bis 12 Kettengliedern versteht, die ein, zwei oder drei Heteroatome ausgewählt aus Sauerstoff, Stickstoff oder Schwefel enthält, wobei es sich versteht, daß die Heteroarylgruppe gegebenenfalls durch eine oder mehrere, gleichartige oder verschiedenartige Gruppen substituiert sein kann, ausgewählt aus:
- Halogen,
- geradkettigem oder verzweigtem (C₁-C₆)-Alkyl, welches gegebenenfalls durch eine Hydroxy-, Carboxy- oder Carbamoylgruppe substituiert ist, wobei die Carbamoylgruppe ihrerseits gegebenenfalls durch eine oder zwei geradkettige oder verzweigte (C₁-C₆)-Alkylgruppen substituiert ist,
- Hydroxy,
- Oxo,
- geradkettigem oder verzweigtem (C₁-C₆)-Alkoxy,
- geradkettigem oder verzweigtem (C₁-C₆)-Trihalogenalkyl,
- Amino, gegebenenfalls N-substituiert durch eine oder zwei geradkettige oder verzweigte (C₁-C₆)-Alkylgruppen,
- Carboxymethoxy
- und Carbamoylmethoxy, gegebenenfalls N-substituiert durch eine oder zwei Gruppen ausgewählt aus geradkettigem oder verzweigtem (C₁-C₆)-Alkyl, geradkettigem oder verzweigtem (C₁-C₆)-Hydroxyalkyl, Alkoxyalkyl, wobei die Alkoxy- und Alkylreste jeweils geradkettige oder verzweigte (C₁-C₆)-Gruppen sind, und Pyridylalkyl, worin der Alkylrest eine geradkettige oder verzweigte (C₁-C₆)-Gruppe ist.

2. Verbindung der Formel (I) nach Anspruch 1, worin die Konfiguration des Asymmetriezentrums in alpha-Stellung zu der Amidgruppe die Konfiguration (S) ist.

3. Verbindung der Formel (I) nach einem der Ansprüche 1 oder 2, worin m den Wert 1 besitzt, ihre Enantiomeren sowie ihre Additionssalze mit einer pharmazeutisch annehmbaren Säure.

4. Verbindung der Formel (I) nach einem der Ansprüche 1 bis 3, worin n den Wert 1 besitzt, ihre Enantiomeren sowie ihre Additionssalze mit einer pharmazeutisch annehmbaren Säure.

5. Verbindung der Formel (I) nach einem der Ansprüche 1 bis 4, worin R₁ ein Wasserstoffatom bedeutet, ihre Enantiomeren sowie ihre Additionssalze mit einer pharmazeutisch annehmbaren Säure.

6. Verbindung der Formel (I) nach einem der Ansprüche 1 bis 5, worin R₂ ein Wasserstoffatom bedeutet, ihre Enantiomeren sowie ihre Additionssalze mit einer pharmazeutisch annehmbaren Säure.

7. Verbindung der Formel (I) nach einem der Ansprüche 1 bis 6, worin R₃ ein Wasserstoffatom bedeutet, ihre Enantiomeren sowie ihre Additionssalze mit einer pharmazeutisch annehmbaren Säure.

8. Verbindung der Formel (I) nach einem der Ansprüche 1 bis 7, worin R₄ und R₅ jeweils ein Wasserstoffatom bedeuten, ihre Enantiomeren sowie ihre Additionssalze mit einer pharmazeutisch annehmbaren Säure.

9. Verbindung der Formel (I) nach einem der Ansprüche 1 bis 7, worin R₄ und R₅ benachbart sind und gemeinsam mit den sie tragenden Kohlenstoffatomen einen Benzoring bilden, ihre Enantiomeren sowie ihre Additionssalze mit einer pharmazeutisch annehmbaren Säure.

10. Verbindung der Formel (I) nach einem der Ansprüche 1 bis 9, worin Ar eine Phenyl-, Thienyl- oder Pyridylgruppe bedeutet, wobei jede dieser Gruppen nicht substituiert ist oder durch eine oder mehrere gleichartige oder verschiedenartige Gruppen substituiert ist, ausgewählt aus:
- Halogen,
- geradkettigem oder verzweigtem (C₁-C₆)-Alkyl, das gegebenenfalls durch eine Hydroxy-, Carboxy- oder Carbamoylgruppe substituiert ist, wobei die Carbamoylgruppe ihrerseits gegebenenfalls durch eine oder zwei geradkettige oder verzweigte (C₁-C₆)-Alkylgruppen substituiert ist,
- geradkettigem oder verzweigtem (C₁-C₆)-Alkoxy,
- Hydroxy,
- geradkettigem oder verzweigtem (C₁-C₆)-Trihalogenalkyl,
- Amino, gegebenenfalls substituiert durch eine oder zwei geradkettige oder verzweigte (C₁-C₆)-Alkylgruppen.
- Carboxymethoxy
- und Carbamoylmethoxy, gegebenenfalls N-substituiert durch eine oder zwei Gruppen ausgewählt aus geradkettigem oder verzweigtem (C₁-C₆)-Alkyl, geradkettigem oder verzweigtem (C₁-C₆)-Hydroxyalkyl, Alkoxyalkyl, wobei die Alkoxy- und Alkylreste jeweils geradkettige oder verzweigte (C₁-C₆)-Gruppen sind, und der Pyridylalkyl, deren Alkylrest eine geradkettige oder verzweigte (C₁-C₆)-Gruppe ist,
ihre Enantiomere sowie ihre Additionssalze mit einer pharmazeutisch annehmbaren Säure.

11. Verbindung der Formel (I) nach Anspruch 10, worin Ar eine Phenylgruppe bedeutet, die nicht substituiert ist oder durch ein oder mehrere gleichartige oder verschiedenartige Halogenatome ausgewählt aus Fluor und Chlor substituiert ist, deren Enantiomere sowie ihre Additionssalze mit einer pharmazeutisch annehmbaren Säure.

12. Verbindung der Formel (I) nach Anspruch 1, ausgewählt aus:
- 3-{[2,2-Difluor-2-(1-oxido-2-pyridyl)-ethyl]-amino}-N-(2-fluorbenzyl)-4-oxo-4,6,7,8-tetrahydropyrrolo[1,2-a]pyrazin-6-carboxamid sowie sein Enantiomeres (6S),
- 3-{[2,2-Difluor-2-(1-oxido-2-pyridyl)-ethyl]-amino}-N-(2,6-difluorbenzyl)-4-oxo-4,6,7,8-tetrahydropyrrolo[1,2-a]pyrazin-6-carboxamid sowie sein Enantiomeres (6S),
- 3-([2,2-Difluor-2-(1-oxido-2-pyridyl)-ethyl]-amino)-N-(2-chlorbenzyl)-4-oxo-4,6,7,8-tetrahydropyrrolo[1,2-a]pyrazin-6-carboxamid sowie sein Enantiomeres (6S),
- 3-{[2,2-Difluor-2-(1-oxido-2-pyridyl)-ethyl]-amino}-N-(2,5-difluorbenzyl)-4-oxo-4,6,7,8-tetrahydropyrrolo[1,2-a]pyrazin-6-carboxamid sowie sein Enantiomeres (6S),
- 3-{[2,2-Difluor-2-(1-oxido-2-pyridyl)-ethyl]-amino}-N-(2,3-difluorbenzyl)-4-oxo-4,6,7,8-tetrahydropyrrolo [1,2-a]pyrazin-6-carboxamid sowie sein Enantiomeres (6S) und
- 3-([2,2-Difluor-2-(1-oxido-2-pyridyl)-ethyl]-amino)-N-(2,3,6-trifluorbenzyl)-4-oxo-4,6,7,8-tetrahydropyrrolo[1,2-a]pyrazin-6-carboxamid sowie sein Enantiomeres (6S).

13. Verfahren zur Herstellung der Verbindungen der Formel (I) nach Anspruch 1, gemäß dem man eine Verbindung der Formel (II): 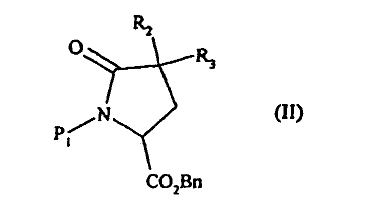 in der R₂ und R₃ die bezüglich der Formel (I) angegebenen Bedeutungen besitzen, P₁ eine Schutzgruppe für die Aminofunktion darstellt und Bn die Benzylgruppe bedeutet,
mit Hilfe eines Reduktionsmittels reduziert
zur Bildung der Verbindung der Formel (III): 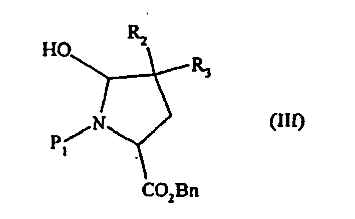 in der R₂, R₃, P₂ und Bn die oben angegebenen Bedeutungen besitzen,
deren Hydroxyfunktion man mit klassischen Reaktionen der organischen Chemie in die Methoxyfunktion und dann in die Cyanofunktion umwandelt, so daß man nach der Abspaltung der Schutzgruppe der Aminofunktion die Verbindung der Formel (IV) erhält: 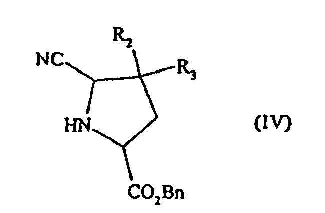 in der R₂, R₃ und Bn die oben angegebenen Bedeutungen besitzen,
welche man mit Oxalylchlorid umsetzt zur Bildung der Verbindung der Formel (V): 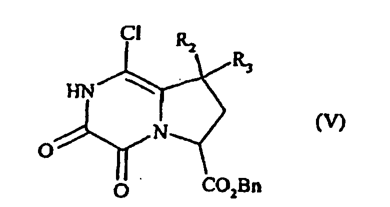 in der R₂, R₃ und Bn die oben angegebenen Bedeutungen besitzen,
welche man einer katalytischen Hydrierungsreaktion unterwirft zur Bildung der Verbindung der Formel (VI): 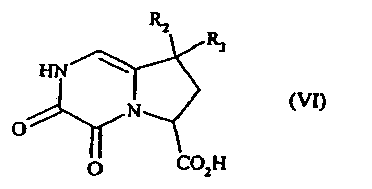 in der R₂ und R₃ die oben angegebenen Bedeutungen besitzen,
welche man zu der Verbindung der Formel (VII) verestert: 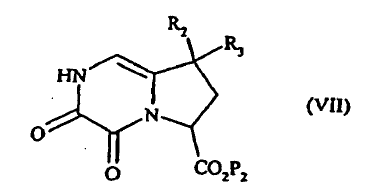 in der R₂ und R₃ die oben angegebenen Bedeutungen besitzen und P₂ eine geradkettige oder verzweigte (C₁-C₆)-Alkylgruppe darstellt,
welche man mit einem Bromierungsmittel umsetzt zur Bildung der Verbindung der Formel (VIII): 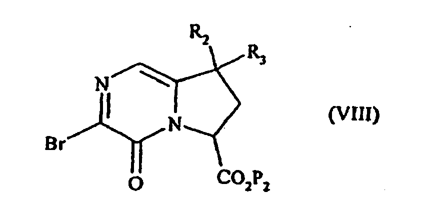 in der R₂, R₃ und P₂ die oben angegebenen Bedeutungen besitzen,
welche man mit 2-Mercaptopyridin umsetzt zur Bildung der Verbindung der Formel (IX): 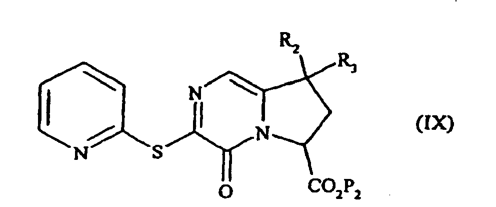 in der R₂, R₃ und P₂ die oben angegebenen Bedeutungen besitzen,
welche man mit dem N-Oxid der Formel (X): 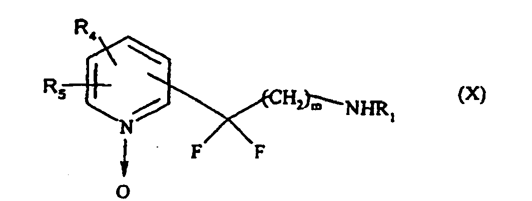 in der m, R₁, R₄ und R₅ die bezüglich der Formel (I) angegebenen Bedeutungen besitzen,
umsetzt zur Bildung der Verbindung der Formel (XI): 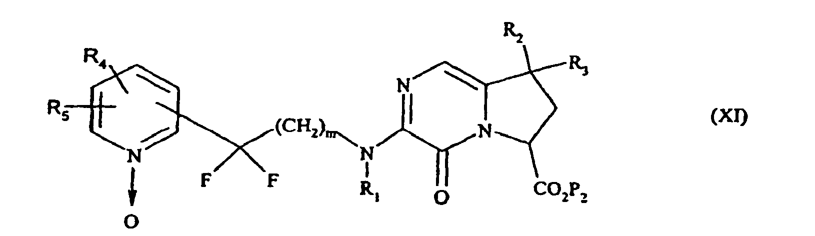 in der m, R₁, R₂, R₃, R₄, R₅ und P₂ die oben angegebenen Bedeutungen besitzen, von der man die Schutzgruppe der Säurefunktion abspaltet zur Bildung der Verbindung der Formel (XII): 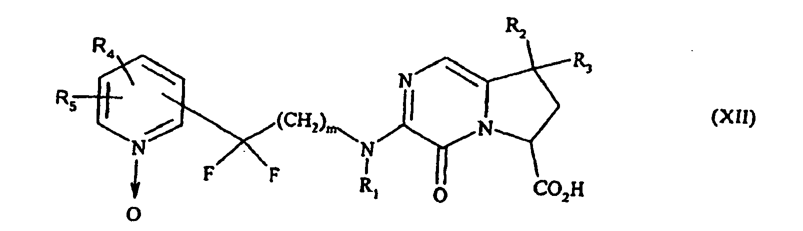 welche man mit einer Verbindung der Formel (XIII): 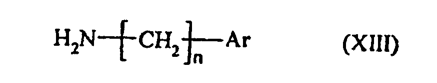 in der n und Ar die bezüglich der Formel (I) angegebenen Bedeutungen besitzen,
in Gegenwart eines Kupplungsmittels umsetzt
zur Bildung der Verbindung der Formel (I).

14. Pharmazeutische Zubereitung enthaltend als Wirkstoff eine Verbindung nach einem der Ansprüche 1 bis 12 in Kombination mit einem oder mehreren inerten, nichttoxischen und pharmazeutisch annehmbaren Hilfsstoffen.

15. Verwendung der Verbindungen der Formel (I) nach einem der Ansprüche 1 bis 12 zur Herstellung von Arzneimitteln, die als Thrombininhibitoren geeignet sind.

16. Verwendung der Verbindungen der Formel (I) nach einem der Ansprüche 1 bis 12 für die Herstellung von Arzneimitteln, die für die Behandlung von stabiler oder nichtstabiler Angina, Erkrankungen thrombotischen Ursprungs und/oder von solchen Krankheiten, die zu thrombotischen Komplikationen Anlaß geben, bei der Behandlung oder der Vorbeugung von Myokardinfarkt und venösen oder arteriellen Thrombosen, sowie zur Behandlung von Komplikationen von vaskulären und kardiovaskulären Erkrankungen, wie Atherosklerose, Arteritis, Venenerkrankungen und bei der Behandlung sämtlicher Erkrankungen, bei denen die Bildung und/oder eine Aktivität von Thrombin beteiligt ist, nützlich sind.
